Europäisches Patentamt

European Patent Office

Office européen des brevets

(11) Publication number: **0 362 179**
**A2**

# EUROPEAN PATENT APPLICATION

(21) Application number: 89870129.7

(22) Date of filing: 24.08.89

(51) Int. Cl.5: **C12N 1/18 , C12N 15/81 ,**
**C12N 15/62 , C12P 21/02 ,**
**C12N 15/49 , C12N 15/25 ,**
**C12N 15/31 , C07K 15/00 ,**
**C12N 15/19 , A61K 39/10 ,**
**//(C12N1/18,C12R1:865)**

(30) Priority: 25.08.88 US 236699

(43) Date of publication of application:
04.04.90 Bulletin 90/14

(84) Designated Contracting States:
AT BE CH DE ES FR GB GR IT LI LU NL SE

(71) Applicant: **SMITHKLINE BEECHAM**
**CORPORATION**
**One Franklin Plaza**
**Philadelphia Pennsylvania 19103(US)**

(72) Inventor: **Bruck, Claudine**
**Avenue du Gris Moulin, 53**
**B-1310 La Hulpe(BE)**
Inventor: **Cohen, Joseph**
**Rue Edmond Picard, 52**
**B-1180 Bruxelles(BE)**
Inventor: **Gorman, Jessica Angel**
**152 Ridgefield Road**
**Newtown Square Pennsylvania 19073(US)**
Inventor: **Koltin, Yigal**
**16B Hashalom Street**
**Hod Hasharon(IL)**
Inventor: **Locht, Camille**
**Avenue Bel Horizon, 40**
**B-1320 Genval (Rixensart)(BE)**
Inventor: **Van Wijnendaele, Frans**
**Teckerstraat, 12**
**B-3052 Ottenburg(BE)**

(74) Representative: **Tasset, Gérard**
**Smithkline Biologicals s.a. Rue de l'Institut**
**89**
**B-1330 Rixensart(BE)**

(54) Recombinant saccharomyces.

(57) A method is described for cloning and expressing DNA sequences derived from Candida in Saccharomyces; including a method for expressing and secreting heterologous gene products under the control of a signal sequence derived from Candida in Saccharomyces. Also provided are a method for characterizing the function of the Candida DNA sequences, a Saccharomyces host microorganism transformed by the DNA sequences, and a recombinant plasmid useful for performing such transformation.

## RECOMBINANT SACCHAROMYCES

This invention is related generally to cloning and expression of DNA sequences from Candida in Saccharomyces. More particularly, the invention relates to the expression of Candida gene sequences in Saccharomyces cells to complement Saccharomyces host cell function and to the expression and secretion of heterologous genes under the control of Candida promoter/signal sequences in Saccharomyces cells. This invention also relates to a vaccine against whooping cough which comprises a truncated B. pertussis S1 subunit protein expressed and secreted in a Saccharomyces cell under the control of Candida promoter/signal sequences.

### Summary of the Invention

One aspect of this invention is a transformed host microorganism of the genus Saccharomyces which comprises a functional DNA sequence derived from a variety of Candida species.

Another aspect of this invention is a recombinant plasmid comprising a replicator region and a functional DNA sequence derived from Candida.

This invention also provides a promoter sequence and a yeast secretary signal sequence derived from the C. albicans glucoamylasegene for use in expression systems in yeast.

Still another aspect of this invention provides a recombinant plasmid comprising a yeast promoter, the signal sequence from the C. albicans glucoamylase gene and a heterologous structural gene, capable upon transformation and expression in Saccharomyces of producing a hybrid fused gene product.

This invention also relates to a method of expressing a functional DNA sequence derived from Candida which comprises transforming a host microorganism of the genus Saccharomyces with the functional DNA sequence, and culturing the transformed host under suitable conditions such that the functional DNA sequence is expressed.

Yet another aspect of this invention is a method of characterizing a DNA sequence derived from Candida by its function which comprises (a) transforming a host microorganism of the genus Saccharomyces with the DNA sequence, wherein said host microorganism lacks a known function, so that the Candida DNA can complement the host microorganism; and (b) assaying for presence of the function in the transformed host microorganism.

This invention also relates to a method of producing a heterologous polypeptide in Saccharomyces which comprises culturing a transformed host microorganism of the genus Saccharomyces in suitable culturing media wherein said host microorganism comprises a promoter and/or signal sequence derived from Candida fused, directly or indirectly, in phase and in proper orientation to a heterologous structural gene which codes for the polypeptide. Where the signal sequence is used in the method, the polypeptide is expressed and secreted from the yeast cell host.

Thus, a further aspect of this invention includes heterologous polypeptides or fragments thereof secreted from Saccharomyces host cells according to this invention. Among these secreted polypeptides is a recombinant HIV-I glycoprotein gp160 unprocessed into its gp120 and gp41 components.

The present invention also relates to a vaccine for stimulating protection against whooping cough, wherein such vaccine comprises an immunoprotective and non-toxic amount of S1-GA protein. Such vaccine may comprise such protein alone or in conjunction with other antigens and/or adjuvants. In addition, the present invention also provides a method for inoculating a human against whooping cough which comprises administering the vaccine of this invention to such human.

### Brief Description of the Drawings

Fig. 1 presents the nucleotide sequence of the promoter and signal sequence of C. albicans glucoamylase gene.

Fig. 2 depicts the expression vector employed in Example 10.

Fig. 3 presents the nucleotide and amino acid sequence of the signal sequence of C. albicans glucoamylase gene.

Fig. 4 presents the nucleotide and amino acid sequence of the synthetic linker employed in Example 11.

Fig. 5 presents the DNA and amino acid sequence of TGF-α.

Detailed Description of the Invention

The transformed host microorganism of the subject invention is any member of the genus Saccharomyces into which a functional DNA sequence derived from Candida has been introduced. Saccharomyces cerevisiae is preferred because it is the most well studied member of the genus and has been the species most frequently employed for recombinant DNA purposes.

The recombinant plasmid of the subject invention comprised a functional DNA sequence derived from any species of the genus Candida including species, Candida albicans, Candida tropicalis, Candida stellatoidea, Candida pseudotropicalis, Candida parapsilosis, Candida quillieromondi, Candida utilus and Candida kruisi. DNA derived from Candida albicans is most preferred because it was the first DNA utilized in the subject invention since it is derived from the genus member most likely to cause candidiasis.

By the term "functional DNA sequence" is meant any discrete region of DNA derived directly or indirectly from Candida which functions in Candida as a complete gene expression unit, a structural gene, a promoter, a signal sequence or a regulatory region. By "complete gene expression unit" is meant a structural gene and the promoter and regulatory regions required for its transcription and translation. By "promoter" is meant any region upstream of a structural gene which permits binding of RNA polymerase and transcription to occur. By "regulatory region" is meant any region which regulates transcription of the gene. By "structural gene" is meant a coding sequence for a polypeptide which serves to be the template for the synthesis of mRNA. By "signal sequence" is meant a region upstream of a structural gene which targets the struction gene to the secretion pathway of the host.

The recombinant plasmid of the subject invention also comprises a replicator region, preferably a Saccharomyces or Candida replicator region. By "replicator region" is meant any DNA fragment, preferably derived directly or indirectly from yeast, such as Saccharomyces or Candida, which can be used to maintain, extrachromosomally, a functional DNA sequence derived from Candida in the host Saccharomyces microorganism. Particularly preferred plasmids are those comprising a Saccharomyces-Escherichia coli shuttle vector such as the one described by Broach et al. Gene, 8:121-133 (1979). Illustrative of vectors which can be used in this invention by cloning therein a functional DNA sequence derived from Candida are the following plasmids which are publicly available, e.g., from the American Type Culture Collection, Rockville, Maryland, U.S.A. (ATCC accession number in parentheses): pBIT-1 (37087), pBTI-7 (37088), pBTI-9 (37089), pBTI-10 (37090), pLC544 (37013), pRB3 (37059), pRB4 (37058), pRB5 (37051), pRB8 (37109), pRB9 (37057), pRB10 (37056), pRB18 (37092), pRB20 (37054), pRB21 (37055), pRB22 (37053), pRB12 (37061), pRB14 (37063), pRB15 (37062), pRB16 (37060), pRB19 (37064), YEp2 (37076), YRp17 (37078), YEp13 (37115).

The Candida DNA sequence can be prepared by known techniques such as the one described by Rigsby et al. Molec. Cell. Biol., 2:853-862 (1982). The isolated Candida DNA can then be further fragmented by treatment with one or more restriction enzymes.

A recombinant plasmid containing a functional Candida DNA sequence and containing an Escherichia coli replicator region can be used to transform Escherichia coli to amplify the plasmid. Such transformation and amplification can be accomplished by known techniques. The recombinant plasmid is then retrieved from E. coli by known techniques.

The retrieved recombinant plasmid can then be used to transform a host microorganism belonging to the genus Saccharomyces. Such transformation can be accomplished by the random insertion of recombinant plasmid DNA fragments into covalently closed circular yeast plasmids and (a) transforming spheroplasts therewith, such as by the method described by Hinnen et al., Proc Nat'l. Acad Sci. USA, 75:1929-1933 (1978); or (b) by using the lithium acetate or lithium chloride method of yeast transformation such as described by Ito et al., J. Bacteriol., 153:163(1983). DNA from Candida can also be transformed into a Saccharomyces microorganism by fusion of Candida and Saccharomyces protoplasts. The introduced Candida DNA can be integrated into the host Saccharomyces microorganism's chromosomal DNA, such as by recombination or can remain extrachromosomal. Thus, yet another exemplary method is transformation of a host Saccharomyces organism directly with Candida DNA which, optionally, has flanking sequences which are homologous to a contiguous sequence in the host's chromosomal DNA thereby permitting recombination with the host's chromosomal DNA.

Promoter regions and signal sequences derived from Candida by the process of the subject invention can be used to express coding sequences from Candida, Saccharomyces or other organisms, viruses or cells in the transformed Saccharomyces host. Such promoters and/or signal sequences can be fused to a heterologous structural gene, such as sequences coding for insulin, interferon, growth hormone bacterial and viral proteins, etc., directly by fusing all or a portion of the promoter's and/or signal sequence's natural nucleotide sequence to the coding sequence for the desired polypeptide, thereby producing a hybrid gene. For example, one such promoter and signal region is derived from C. albicans glucoamylase gene. This functional DNA sequence when inserted in a plasmid and fused to a heterologous structural gene enables the expression and secretion of a hybrid fusion protein. An alternative expression system employs another yeast promoter and the C. albicans glucoamylase gene signal sequence for expression and secretion of a desired fusion gene. When Saccharomyces host microorganisms, which have been transformed by a plasmid containing such hybrid gene, are cultured in suitable culturing media they produce the polypeptide. Under control of the appropriate promoter and signal sequence, the polypeptide may be secreted into the culture medium.

In addition to those Candida promoters disclosed herein, additional promoters can be discovered by cloning random fragments of Candida DNA in Saccharomyces plasmids designed for promoter selection. Such plasmids can be designed by known techniques such as the one described by Casadaban et al., Methods in Enzymology, 100: 293-308(1983).

Structural genes derived from Candida can be expressed in Saccharomyces from their natural Candida promoters, from heterologous promoters derived from Candida or Saccharomyces, or from other functional heterologous promoters which are derived from other organisms or produced from synthetic oligonucleotides and which function in Saccharomyces. Similarly, structural genes of non-Candida origin may be expressed from a variety of promoters including the glucoamylase gene promoter with its signal sequence.

Many known Saccharomyces mutants are defective in defined functions, such as a specific step in leucine or histidine biosynthesis. This function could be supplied if such mutants were transformed with DNA sequences derived from Candida which contain genes coding for such functions, Where the function to be characterized is not already defective in an available Saccharomyces strain, such mutations can be induced by known techniques, such as by ultraviolet radiation, chemical mutagenesis, or genetic manipulation. In some cases the function displayed by the transformed host microorganism will be one which is naturally not present in Saccharomyces. For example, a host Saccharomyces cerevisiae microorganism can be transformed by a functional DNA sequence according to the method of this invention to display functions it does not naturally possess such as sorbitol utilization, maltose utilization by isomaltase catalyzed cleavage of $\alpha$-1-6-glucosidic bonds (such as $\alpha$-methyl-D-glucopyranoside hydrolysis), soluble starch utilization by glucoamylase catalyzed cleavage of $\alpha$-1-4-glucosidic bonds or resistance to an anti-fungal absent.

The development of a genomic library covering, the entire genome of Candida enables the characterization of the functions of the DNA derived from Candida. This will permit the genetics of this asexual pathogen to be more closely analyzed than has been possible in the past and to facilitate the development of anti-candidial agents. The preferred method of characterizing the functions of DNA derived from Candida albicans in Saccharomyces cerevisiae so that the Candida genes can be studied in the background of a well characterized eukaryotic organism. Thus, the genetics of Candida albicans can be studied by isolation of genes via complementation of functions displayed by transformed Saccharomyces cerevisiae. The abundance of available Saccharomyces cerevisiae mutant strains, together with the possibility of selection of additional mutants, makes it possible to effect such complementation. Therefore, it is possible to select for Candida albicans genes associated with any one of a number of functions, such as those associated with defined biochemical reactions, or those involved in cell division or cell structure. The availability of the genomic library and a system for the identification of the selected genes offers a new approach to resolve some of the basic differences that make one organism economically useful and another organism a serious pathogen.

Using the method of this invention plasmids carrying one or more functional C. albicans genes which complement a Saccharomyces mutation or confer a new characteristic to the Saccharomyces strain transformed therewith can be isolated and characterized. Such functional C. albicans genes can include a gene complementing the S. cerevisiae trpl mutation, a gene complementing the S. cerevisiae his3 mutation, a gene conferring high level resistance to the anti-fungal absent benomyl, a gene allowing for growth on sorbitol, a gene allowing for growth on isomaltase, a gene allowing for growth on soluble starch (i.e., a glucoamylase gene), and a gene encoding the enzyme galactokinase which complements a S. cerevisiae gall mutation. Such genes are useful as markers for isolating Saccharomyces hosts successfully transformed with plasmids containing such genes. Each of the promoters of the C. albicans sorbitol, isomaltase and soluble starch utilization genes is also useful as a regulatable promoter for a Saccharomyces microorganism transformed with a plasmid containing such a promoter. Plasmids containing C. albicans functional DNA sequences are useful for production in Saccharomyces of heterologous gene products, such as pharmaceuticals, vaccinal antigens and hormones.

Also according to a method of this invention, a heterologous gene encoding, e.g. an HIV-1 surface glycoprotein (gp160), a B. pertussis toxin subunit (S1), Transforming Growth Factor-alpha (TGF-α) and Interleukin 1β (IL1-β) may be expressed and secreted in Saccharomyces under control of a yeast promoter and the signal sequence of the C. albicans glucoamylase gene. Plasmids containing a selected yeast promoter, the glucoamylase signal sequence and the heterologous structural gene may be transformed into a selected Saccharomyces yeast cell. A gene product is expressed thereby as a hybrid or fusion protein or polypeptide and is processed and secreted into the culture medium. Secretion of the fusion protein enables purification of the protein to be greatly simplified and allows post-translational modifications of the protein, e.g. glycosylation, to be effected.

This invention also relates to a vaccine against whooping cough (pertussis). Pertussis toxin is a protein exotoxin of B. pertussis which plays a major role in the pathogenesis of whooping cough and is believed to be the major protective antigen of B. pertussis. [See, A.A. Weiss et al., Ann. Rev. Microbiol., 40:661 (1986)]. Pertussis toxin is a hexameric protein composed of five dissimilar subunits called S1 through S5 according to their decreasing molecular weights. [See, M. Tamura et al., Biochem., 21:5516 (1982)]. Pertussis toxin is structured in an A-B model in which the B-oligomer (subunits S2 through S5) is responsible for the binding of the toxin to its receptor in the target cell membranes, and the A-protomer (S1 subunit) contains enzymatic activity.

The nucleotide coding sequence of the entire pertussis toxin operon has been determined. See, e.g., Keith, et al., United States Patent Application Serial No. (U.S.S.N.) 843,727, filed March 26, 1986, the entire disclosure of which is hereby incorporated by reference. Furthermore DNA containing the pertussis toxin coding sequence may be isolated from any publicly available B. pertussis strain. For example. various strains of B. pertussis are publicly available from commercial depositories, e.g., from the American Type Culture Collection, Rockville, Maryland U.S.A. Such isolation may be effected by the method of Keith, et al., U.S.S.N. 843,727, supra, or any other conventional method.

The present invention is based on the the discovery that S1-GA protein has the ability to elicit a protective immunogenic response. Thus, the present invention relates to S1-GA protein, the S1-GA protein coding sequence, a vaccine for stimulating protection against whooping cough, wherein such vaccine comprises an immunoprotective and non-toxic amount of S1-GA protein. Such vaccine may comprise such protein alone or in conjunction with other antigens and/or adjuvants. In addition, the present invention also provides a method for inoculating a human against whooping cough which comprises administering the vaccine of this invention to such human.

By the term 'immunoprotective amount' as used herein is meant an amount of S1-GA protein which will elicit a sufficient protective immune response against pertussis after such amount of such protein is administered to a human host.

By the term 'S1-GA protein' is meant a soluble form of the pertussis toxin (PT) S1 subunit protein which has been expressed by a host Saccharomyces cell transformed by a recombinant DNA fragment comprising 186 amino acids of the S1 subunit coding sequence (amino acids 2 to 187 of the N-terminal of the mature S1 protein) fused, in phase, to (a) the Candida albicans glucoamylase signal peptide coding sequence or (b) somewhere within the Candida albicans glucoamylase gene provided that such gene includes its signal peptide coding region, and any functional mutant or derivative thereof. The creation of the rS1d gene and its expression under the lacZ transcriptional and translational control on plasmid pTXS11 is described in full in Locht et al., Infect. Immun., 55, 963-967 (1987), the entire disclosure of which is hereby incorporated by reference.

By the term 'functional mutant and derivative' is meant any protein encoded by any mutation or derivation of the S1-GA protein coding sequence which, upon expression in a host cell, e.g., a Saccharomyces host cell results in a protein which retains the biological activity of the S1-GA protein, e.g., solubility and a protective immunogenic response against pertussis. Production of such mutants and derivatives is within the routine skill of the art. Such functional mutants and derivatives of the S1-GA coding sequence include chemically modified forms thereof, e.g., glycosylated and alkylated forms which have substantially the same biological activity as S1-GA protein. They also include mutants and derivatives in which one or more amino acids have been rearranged, added, deleted or substituted or in which a heterologous polypeptide has been fused or conjugated thereto. Thus, by the term 'functional mutants and derivatives of the S1-GA protein coding sequence' is meant any coding sequence, such as a fusion of the S1-GA protein coding sequence to another protein's coding sequence, as well as a S1-GA protein coding sequence encompassing substitutions, deletions or additions of amino acids, which encodes a protein which has substantially the same biological activity as native S1-GA protein. Such functional mutants and

5

derivatives are conventionally prepared by one of skill in the art employing, e.g., site directed mutagenesis, random mutagenesis, chemical synthesis of nucleotide or peptide sequences, truncation or deletion of part of the coding sequence for native S1-GA protein, chemical modification of the above or concatenation of more than one segment of the S1-GA protein coding sequence. Such functional mutants and derivatives of the S1-GA protein coding sequence also include chemically modified forms thereof, e.g., glycosylated and alkylated forms which have substantially the same biological activity as native S1-GA protein.

This invention is also related to a recombinant DNA molecule, such as a cDNA, which contains the S1-GA protein coding sequence. Such cDNA can be prepared by conventional techniques such as by reverse transcription of mRNA isolated by conventional techniques from a cell or culture producing S1-GA protein, such as by the method of Han et al., Biochemistry, 26, 1617-1625 (1987). As a further embodiment of the recombinant DNA molecule of the invention there is provided a recombinant DNA vector comprising the coding sequence of this invention. This vector preferably contains the above coding sequence in operative association with suitable expression control sequences. By 'suitable expression control sequences' is meant those sequences which regulate the transcription and translation of a structural gene. Such expression control sequences are well known in the art.

Still a further aspect of this invention is a host cell transformed with the vector of this invention. Such host cell is capable of growth in a culture medium and is capable of expressing the coding sequence of the invention. Such host cell is prepared by the method of this invention, i.e., transforming a desired host cell with the vector of the invention. Such transformation is accomplished by utilizing conventional transformation techniques. Host cells which may be suitable include, but are not limited to, mammalian cells insect cells, bacterial cells, plant cells and fungal cells. Thus, this invention is not limited to any specific host cels, although a Saccharomyces host cell is preferred.

The present invention also relates to a method of producing the protein encoded by the S1-GA coding sequence of this invention which comprises culturing the transformed host of the invention in appropriate culture media and isolating such protein. By "appropriate culture media" is meant that media which will enable the transformed host to survive and which will also enable such host to express the coding sequence of the invention in recoverable quantity. It will be appreciated by one of skill in the art that the appropriate culture media to use will depend upon the host cell employed. The isolation of the protein so produced is accomplished from a culture lysate of the host or, if appropriate, directly from the host's culture medium and is carried out by conventional protein isolation techniques such as those discussed above.

This invention also relates to a synthetic DNA molecule containing the S1-GA protein coding sequence. By the term 'synthetic' is meant a DNA molecule prepared by synthetic rather than recombinant techniques. Such techniques are well known in the art using commercially available DNA synthesizers.

The vaccine of this invention comprises an immunoprotective and non-toxic amount of S1-GA protein. Such vaccine preferably contains 5-25 $\mu$g of such S1-GA protein but is not limited to these amounts.

Other antigens which are known to be desirably administered in conjunction with pertussis toxin may also be included in the vaccine of this invention. Such additional components are known to those of skill in the art. Preferable additional components include tetanus toxoid and/or diphtheria toxoid as well as filamentous haemagglutinin (FHA) and/or any other protective antigen of B. pertussis.

The provision of such a vaccine thus allows another aspect of the present invention, i.e., a method for immunizing a human against pertussis which comprises administering the vaccine of the subject invention to such human.

The mode of administration of the vaccine of the invention may be any suitable route which delivers an immunoprotective amount of the protein of the subject invention to the host. However, the vaccine is preferably administered parenterally via the intramuscular or deep subcutaneous routes. Other modes of administration may also be employed, where desired, such as oral administration or via other parenteral routes, i.e., intradermally, intranasally, or intravenously.

The vaccine of the invention may be prepared as a pharmaceutical composition containing an immunoprotective and non-toxic amount of S1-GA protein in a nontoxic and sterile pharmaceutically acceptable carrier. Where the administration of the vaccine is parenterally, the protein comprised by the vaccine can be optionally admixed or absorbed with any conventional adjuvant, for use as a non-specific irritant to attract or enhance an immune response. Such adjuvants include, among others, aluminum hydroxide, aluminum phosphate, muramyl dipeptide and saponins, such as Quil A. As a further exemplary alternative of the preparation of the vaccine of the invention, the protein comprised by the vaccine can be encapsulated within microparticles, such as liposomes. In yet another exemplary alternative of the preparation of the vaccine of the invention, the protein comprised by the vaccine can be administered with an immunostimulating macromolecule, or a tetanus toxoid. Alternatively, an aqueous suspension or solution containing the protein of vaccine of the invention, preferably buffered at physiological pH, may be designed for oral ingestion.

It is preferred that the vaccine of the invention, when in a pharmaceutical preparation, be present in unit dosage forms. The appropriate therapeutically effective dose can be readily determined by those of skill in the art. The effective amount of S1-GA protein contained in the vaccine of this invention may be in the range of the effective amounts of antigen in conventional B. pertussis acellular or component vaccines, i.e., 5-25 $\mu$g of protein. This dose may optionally be delivered with various amounts of filamentous haemagglutinin (FHA) (about 10 ug-25 ug) and/or agglutinogens or other antigens. It will be understood, however, that the specific dose level for any particular patient will depend upon a variety of factors including the age, general health, sex, and diet of the patient; the time of administration; the route of administration; synergistic effects with any other drugs being administered; and the degree of protection being sought. Of course, the administration can be repeated at suitable intervals if necessary. Preferably, three doses will be administered, with 3 to 12 months intervals between each dose, with an optional booster dose later in time.

In the following examples, specific embodiments of the invention are more fully disclosed. Examples 1-7 illustrate the expression of Candida genes in Saccharomyces to complement the functions of the native Saccharomyces host cell. Examples 8 and 9 illustrate the isolation and use of Candida replicator regions to control expression of a Candida gene in Saccharomyces. Examples 10-13 illustrate the use of a Candida signal sequence as part of the expression and secretion system for other non-yeast heterologous genes in Saccharomyces. Examples 13 and 14 relate to the vaccine of the invention. These examples are intended to be illustrative of the subject invention and should not be construed as limiting its scope.

## EXAMPLE 1 - ISOLATION OF DNA FROM C. ALBICANS

### A. Construction of the genomic library of C. albicans

The DNA from C. albicans strain 792-1 (courtesy of J. Kwon-Chung, Natl. Inst. Health, U.S.A.) was isolated by the procedure described by Rigsby et al., cited above. The DNA purified by this procedure was on the average 100 kilobase paris (kbp) in length.

A genomic library was constructed in the Saccharomyces-Escherichia coli shuttle vector, YEp13 [ATCC 37115], described by Broach et al., cited above. Calculation of the size of the genome of C. albicans based on the data provided by Rigsby et al., cited above, suggests that the genome size of C. albicans is about 2 x 10⁴ kbp. Therefore, some 8000 independent transformants carrying an insert of 10 kbp are required to provide a genomic library with a probability of 99% that each of the sequences of C. albicans will be represented at least once in this library. This was calculated by the method disclosed by Maniatis et al., "Molecular Cloning - A Laboratory Manual", Cold Spring Harbor Laboratory (1982).

The DNA was partially digested with the restriction endonuclease Sau3A and fractionated by centrifugation in a 5-20% sucrose gradient. The fractions containing DNA fragments in the range of 10 kbp were pooled and used for ligation. Plasmid YEp13 DNA was cleaved at the unique BamHI site and then treated with alkaline phosphatase to prevent self-ligation. Equal concentrations (200 ng. in a 10 $\mu$l reaction mixture) of C. albicans and YEp13 DNA were ligated.

E. coli strains MC1061 (Casadaban et al., J. Mol. Biol., 138:179-207 (1980)) and MB101 (Bolivar et al., Gene, 2:95-113 (1979) were transformed with the ligated mixture according to Lederberg, and Cohen, J. Bacteriol., 119:1072-1074 (1974), With the modification that a 45 min. expression time at 37°C in Luria Broth (LB) was allowed. An estimate of the number of independent transformants was obtained by plating a small sample on LB plates containing 50 $\mu$g/ml of ampicillin. Simultaneously, the remaining sample was inoculated into LB broth containing 50 $\mu$g/ml of ampicillin and incubated overnight to use for DNA isolation. The plate results indicated that some 16,000 independent clones were obtained. To estimate the percentage of transformants which contained inserts, 100 transformants were tested for their resistance to ampicillin and to tetracycline. Since the BamHI site is in the tetracycline resistance gene (tet^R), transformants that contain plasmids with the inserted DNA should be sensitive to tetracycline and resistant to ampicillin, while transformants containing plasmids without inserts should be resistant to both antibiotics. The results obtained indicated that all the transformants were sensitive to tetracycline and resistant to ampicillin suggesting that at least 99% of the transformants were carrying plasmids containing inserted DNA of C. albicans.

DNA was prepared from the overnight LB-ampicillin culture of E. coli, either small scale or large scale by the method of Maniatis et al., cited above, using an aliquot of this original culture as inoculum.

EP 0 362 179 A2

B. Expression of C. albicans genes in S. cerevisiae

(1) General methodology

To isolate cloned DNA sequences of C. albicans by expression in S. cerevisiae, genetically defined haploid strains of S. cerevisiae were employed as hosts for DNA mediated transformation Transformation was performed a) by the spheroplast procedure, as described by Sherman et al., "Methods in Yeast Genetics Laboratory Manual", Cold Spring Harbor Laboratory (1982), or by the lithium chloride or lithium acetate method described by Ito et al., cited above. The yeast were plated on minimal medium (Yeast nitrogen base-Difco, YNB) containing all necessary supplements except one.

Transformation of a defined S. cerevisiae mutant with a plasmid containing the wild type allele of the specific mutation allows the transformed host to grow under conditions restrictive to the non-transformed mutant. In the examples following, the LEU2 gene was used as the first selectable marker. The transformed yeast were then subcultured onto additional types of media to select transformants that expressed other functions original lacking in the S. cerevisiae host strain.

The plasmids were recovered from S. cerevisiae by making a DNA preparation of the desired transformant, using the procedure of Sherman et al., cited above, and using this DNA to transform E. coli by known techniques and used for further analysis. Such further study included: a) transformation of yeast to confirm the presence of a gene conferring the same phenotype as found in the original transformed yeast cell; b) hybridization analysis to confirm the presence of DNA of C. albicans in the hybrid plasmid; and c) further analysis of the C. albicans DNA fragment both structurally and functionally.

(2) Isolation of a Candida albicans gene that complements the trpl mutation of S. cerevisiae.

Spheroplasts of S. cerevisiae strain DBY746 (α, his3-1 leu2-3, leu2-112, ura3-52, trpl-289) were transformed with DNA isolated from the C. albicans library. S. cerevisiae strain DBY746 was obtained from Yeast Genetic Stock Center, Department of Biophysics and Medical Physics, University of California, Berkeley, California, U.S.A. The cells were plated on minimal medium (Difco YNB) containing all required supplements except leucine. The transformed yeast cells expressed the plasmid encoded LEU2 gene. The Leu⁺ cells were harvested from these plates and plated onto minimal medium lacking both leucine and tryptophan. Of the 500 transformants examined, two grew on these plates.

To test whether the growth of these two transformants on medium lacking leucine and tryptophan was due to the presence of a C. albicans DNA insert, rather than due to the reversion of the trpl mutation, the transformants were grown in non-selective yeast extract peptone dextrose (YEPD) medium for 10 generations, and then plated on minimal medium containing all supplements. The colonies were then replica plated onto minimal medium lacking only tryptophan or only leucine.

In all cases, colonies arising from cells that had lost the plasmid i.e., were Leu, also required tryptophan indicating co-segregation of the LEU2 and TRP1 alleles.

Plasmids were recovered from the two transformed isolates by transformation of E. coli HB101 with small yeast DNA preparations, prepared by the method of Sherman et al., cited above, selecting for ampicillin resistant transformants. The transformed E. coli cells were tetracycline sensitive, indicating that the plasmid contained an insert in the tet^R gene. The two plasmids were designated pAR84-1 and pAR84-2.

Plasmid DNA of each was prepared by the method of Maniatis et al., cited above, and used to transform S. cerevisiae strain DBY746. The selection of the transformed cells were performed on medium devoid of leucine but containing the supplements histidine, tryptophan and uracil. The Leu⁺ transformed cells were tested for prototrophy for tryptophan. Between 88 to 100% of the transformants expressed the unselected marker TRP⁺ confirming that both pAR84-1 and pAR82-2 contained a gene complementing the S. cerevisiae trpl mutation and indicating that a functional DNA sequence derived from Candida can be expressed in Saccharomyces.

C. Further Characterization of the Plasmids

To test for specific hybridization of the DNA insert in the recombinant plasmids to genomic DNA of C. albicans, DNA from non-transformed and transformed S. cerevisiae strain DBY746 was isolated according to the procedure described by Sherman et al., cited above, for small scale preparations. The DNA of E. coli

8

with or without plasmid, was also prepared by the method of Maniatis et al., cited above.

The DNA samples were digested with the restriction enzymes HindIII and Bg1II. Digested DNA of C. albicans, strain 792-1, S. cerevisiae strain DBY746, plasmid YEp13, and plasmid pAR84-2 was electrophoresed on agarose gels, and transferred to a nitrocellulose filter by the procedure of Southern, J. Mol Biol., 98:503-517 (1975). The filter was then hybridized sequentially, first with $^{32}$P labelled YEp13 DNA to test whether the vector used hybridized to the genomic DNA of C. albicans, and, second, with $^{32}$P labelled pAR84-2 DNA to determine whether the new plasmid hybridized to the genomic DNA of C. albicans.

The nitrocellulose filters were prehybridized for 3 hrs. at 65°C with a solution containing 6 x SSC and 3 x Denhardt (1 x Denhardt is 0.02% Ficol 400, 0.02% polyvinylpyrrolidone 360 and 0.02% bovine serum albumin) according to the method of Maniatis et al. cited above. Hybridization solution was the same except the $^{32}$P labelled DNA probe prepared by nick translation according to the method of Maniatis et al., cited above, was added. Hybridization was performed for 18 hours at 65°C. After hybridization the filters were washed with 2 x SSC at 65°C. The filters were then exposed to X-ray film.

YEp13 DNA hybridized only to the genomic DNA of S. cerevisiae, whereas pAR84-2 DNA also hybridized to the genomic DNA of C. albicans. The hybridization of pAR84-2 to the S. cerevisiae genomic DNA is due to the S. cerevisiae LEU2 gene that is a part of the plasmid. The hybridization to C. albicans is due to homology of Candida insert with genomic sequences of Candida.

The size of the inserts in each plasmid was estimated by electrophoresis of restriction digests on agarose gels. pAR84-1 contains a 15 kbp fragment of C. albicans DNA, while pAR84-2 contains a 10.4 kbp fragment.

Using the same methodology as that used for the isolation of a C. albicans DNA fragment that complements the S. cerevisiae trpl mutation, plasmids carrying other functional C. albicans genes have been isolated and characterized as described in the following Examples 2-7.

## EXAMPLE 2 - COMPLEMENTATION OF THE S. CEREVISIAE his3 GENE WITH C. ALBICANS DNA

The his3 of S. cerevisiae encodes imidazoleglycerol phosphate dehydrogenase. The isolation of a C. albicans gene that complements the his3 mutation of S. cerevisiae was carried out according to the procedure of Example 1. The Leu$^+$ yeast Transformants were plated on medium lacking leucine and histidine. Colonies which arose were selected.

Plasmids containing a C. albicans gene that complements the his3 mutation of S. cerevisiae were recovered from the selected colonies, described above, by transformation of E. coli HB101 according to the method of Example 1. Plasmid DNA was isolated from the E. coli transformants according to the method of Example 1, and was designated pAR84-3.

Plasmid pAR84-3, containing the C. albicans gene that complements the his3 mutation of S. cerevisiae was transformed into S. cerevisiae strain DBY746 according to the procedure of Example 1.

Host S. cerevisiae microorganisms were transformed with plasmid pAR84-3 and selected according to the methods described above. These microorganisms were then tested for expression of the his3 gene.

## EXAMPLE 3 - EXPRESSION OF THE C. ALBICANS BENOMYL RESISTANCE GENE IN S. CEREVISIAE

The C. albicans benomyl resistance gene confers high level resistance to the anti-fungal agent benomyl (methyl 1-(butylcarbamoyl)-2-benzimidazole-carbamate). Strains of C. albicans are more resistant to benomyl than S. cerevisiae. This gene thus confers a new characteristic to a transformed S. cerevisiae strain, as follows.

The isolation of the C. albicans benomyl resistance gene was carried out according to the procedure of Example 1, except that S. cerevisiae strain JWG2-2C (α, leu2-3, leu2-112) was employed as the host. S. cerevisiae strain JWG2-2C was derived from a cross of S. cerevisiae strain DC5 (leu2-3, leu2-112, his3, gal2, can1-11), with S. cerevisiae strain YM146 (α, gall $^\triangle$152, trpl-289, ura3-52, rho). S. cerevisiae strain DC5 was obtained from James R. Broach, S.U.N.Y., Stonybrook, New York, U.S.A. S. cerevisiae strain YM146 was obtained from Mark Johnson, Washington State University, Pullman, Washington, U.S.A. The Leu$^+$ transformed cells were plated on mimimal medium (Difco YNB) containing all required supplements except leucine, and also containing benomyl (50) μg/ml). Several benomyl resistant colonies were obtained.

Plasmids containing C. albicans gene that codes for an enzyme conferring benomyl resistance were recovered according to the procedure of Example 1. Plasmid DNA was isolated from individual E. coli transformants according to the small scale method of Sherman et al., cited above. The size of the C.

albicans insert in the recovered plasmids was estimated by agarose gel electrophoresis.

The plasmid DNA containing the C. albicans benomyl resistance gene was isolated from the E. coli transformants, and used to transform S. cerevisiae strain JWG2-11D (α, his3, trpl-289, leu2-3, leu2-112, gall $^\triangle$152) according to the procedure of Example 1. S. cerevisiae strain JWG2-11D was derived by the same method as S. cerevisiae strain JWG2-2C.

Host S. cerevisiae microorganisms, transformed with a plasmid containing the C. albicans benomyl resistance gene according to the method described above, were selected as described above. The presence of plasmid in the transformed hosts was confirmed according to the method of Example 1 by plating on medium containing benomyl. Three independent isolates, designated pCAben17, pCAben3 and pCAben15, were further analyzed by restriction mapping, and all were found to contain an identical 5.8 kilobase pair (kbp) DNA fragment from C. albicans.


## EXAMPLE 4 - EXPRESSION OF THE C. ALBICANS SORBITOL UTILIZATION GENE IN S. CEREVISIAE

The C. albicans sorbitol utilization gene allows for growth on the carbon source sorbitol. Strains of C. albicans can grow on sorbitol but S. cerevisiae and other species of Saccharomyces cannot utilize this sugar. This gene thus confers a new characteristic to the transformed S. cerevisiae strain, as follows.

The isolation of the C. albicans sorbitol utilization gene was carried out according to the procedure of Example 1, except that S. cerevisiae strain JWG2-2C was employed as the host. Leu$^+$ transformants were plated on minimal medium (Difco YNB) containing all required supplements except leucine and glucose, and also containing 2% sorbitol.

Plasmids containing a C. albicans gene that codes for an enzyme conferring sorbitol utilization ability were recovered according to the procedure for Example 1. Plasmid DNA was isolated from individual E. coli transformants according to the small scale method of Sherman et al., cited above, and the size of the C. albicans insert of the recovered plasmids was estimated by agarose gel electrophoresis.

Plasmid DNA containing the C. albicans sorbitol utilization gene was isolated from the E. coli transformants described above, and used to transform S. cerevisiae strain JWG2-11D according to the method of Example 1.

Host cells of S. cerevisiae transformed with a plasmid containing the C. albicans sorbitol utilization gene were selected as described above. The presence of the plasmid in the transformed hosts was confirmed according to the method of Example 1. DNA of two independent isolates, designated pCAsor2 and pCAsor9, were further analyzed by restriction mapping. pCAsor2 was found to contain a 4.5 kbp DNA insert from C. albicans, while pCAsor9 was found to contain a 4.8 kbp DNA insert from C. albicans which showed little homology to the pCAsor2 C. albicans DNA insert.


## EXAMPLE 5 - EXPRESSION OF THE C. ALBICANS GALACTOKINASE GENE IN S. CEREVISIAE

The isolation of the C. albicans galactokinase gene, which complements a S. cerevisiae gall mutation, was carried out according to the procedure of Example 1, except that S. cerevisiae strain JWG2-11D was employed as the host. Leu$^+$ transformants were plated on minimal media (Difco YNB) containing all required supplements except leucine and glucose, and also containing 2% galactose.

Plasmids containing the C. albicans gene that codes for galactokinase were recovered in E. coli according to the procedure for Example 1. Plasmid DNA was isolated from individual E. coli transformants according to the method of Example 1.

The plasmid DNA containing the C. albicans galactokinase gene was isolated from the E. coli transformants as described above and used to transform S. cerevisiae strain JWG2-11D according to the method of Example 1.

Host S. cerevisiae microorganisms, transformed with a plasmid containing the C. albicans galactokinase gene were selected as described above, and the presence of the plasmid in the transformed host was confirmed according to the method of Example 1. Two independent isolates, designated as pCAgall-1 and pCAgall-4 were further analyzed by restriction mapping which revealed that the 7 kbp C. albicans DNA insert in pCAgall-1 is identical to the internal portion of the 13 kbp insert in pCAgall-4.

The presence of a C. albicans DNA sequence encoding galactokinase in the S. cerevisiae transformants was further demonstrated by measuring the level of galactokinase in strain JWG2-11D with or without plasmid pCAgall-4. Enzyme activity measured by the method of Butt et al., PNAS-USA, 81, 3332 3336 (1984), was only detected in galactose induced cultures containing pCAgall-4.

The promoter of the C. albicans galactokinase gene was demonstrated to be positively activated by the S. cerevisiae positive regulator, the GAL-4 gene product, as follows. Strain JWG2-11D containing pCAgall-4 ws mated to another S. cerevisiae strain (genotype: α-trpl-289, leu2-3, leu2-112, gall-152, ura3-52) containing a 2 μm Leu2 shuttle vector containing the S. cerevisiae GAL-4 gene. The resulting diploid was maintained on medium lacking both leucine and tryptophan. The induced level of galactokinase was significantly higher in the presence of the plasmid expressing the GAL-4 gene. This result indicates that the C. albicans sequence in pCAgall-4 contains a galactose requlated promoter which is responsive to S. cerevisiae regulatory proteins.

EXAMPLE 6 - EXPRESSION OF THE C. ALBICANS ISOMALTASE GENE IN S. CEREVISIAE

The enzyme isomaltase cleaves α-1-6-glucosidic bonds, and enables microorganisms expressing it to grow on the carbon source known as α-methyl-D-glucopyranoside (e.g. isomaltase). Strains of C. albicans can grow on this substrate but most strains of S. cerevisiae cannot naturally utilize this sugar. This enzyme has been found in other species of Saccharomyces. Thus, the C. albicans gene which codes for isomaltase confers a new characteristic to the transformed S. cerevisiae host.

The isolation of the C. albicans isomaltase gene was carried out according to the procedure of Example 1. Leu+ transformants were plated on minimal media (Difco YNB) containing all required supplements except leucine, and also containing 2% α-methyl-D-glucopyranoside.

Plasmids containing the C. albicans gene that codes for isomaltase were recovered according to the procedure of Example 1. Plasmid DNA was isolated from individual E. coli transformants according to the method of Example 1.

The plasmid DNA containing the C. albicans isomaltase gene was isolated from the E. coli transformants described above and used to transform S. cerevisiae strain JWG2-11D according to the method of Example 1.

Host S. cerevisiae microorganisms, transformed with a plasmid containing the C. albicans isomaltase gene were selected as described above, and the presence of the plasmid in the transformed host was confirmed according to the method of Example 1.

EXAMPLE 7 - EXPRESSION OF THE C. ALBICANS GLUCOAMYLASE GENE IN S. CEREVISIAE

The C. albicans glucoamylase gene codes for an enzyme allowing for growth on soluble starch. Strains of C. albicans can utilize such soluble starch as a substrate, but S. cerevisiae cannot naturally utilize this carbon source. This enzyme, glucoamylase, has been found in other species of Saccharomyces. Thus, the C. albicans gene which codes for glucoamylase (which cleaves α-1-4 glucosidic bonds) confers a new characteristic to the transformed S. cerevisiae host.

The isolation of the C. albicans glucoamylase gene was carried out substantially according to the procedure of Example 1, except that S. cerevisiae strain JWG2-2C was employed as the host Leu+ transformants were plated on minimal media (Difco YNB) containing all required supplements except leucine and glucose, and also containing 2% soluble starch. The E. coli transformants were probably secreting the glucoamylase since satellite colonies began to grow adjacent to the original large colonies.

Plasmids containing the C. albicans glucoamylase gene were recovered in E. coli and plasmid DNA was isolated from individual E. coli transformants according to the methods of Example 1.

Plasmid DNA containing the C. albicans glucoamylase gene isolated from the E. coli transformants as described above, was used to transform S. cerevisiae strain JWG2-11D according to the method of Example 1.

Host S. cerevisiae microorganisms, transformed with a plasmid according to the method described above, were selected as described above. The presence of the plasmid in the transformed host was confirmed according to the method of Example 1. Three independent isolates, designated as pSTA27-1, pSTA1-3 and pSTA15-1, were further analyzed by restriction mapping which revealed that pSTA27-1 and pSTA1-3 contain an identical C. albicans DNA insert while pSTA15-1 contains a possibly different C. albicans DNA insert.

Plasmid pSTA27-1 was introduced by transformation (Ito et al., cited above) into three different S. cerevisiae strains, one strain (genotype: α, leu2−, his1), a second strain (genotype: α, leu2, his1) and a third strain (genotype: α, leu2, his1). The same three strains were also transformed with the parent vector plasmid YEp13 lacking any C. albicans sequences. The growth of all six transformed strains was tested on complete (YEP) medium containing 0.5% maltooligossacharides (Mos) (Pfanstiel - Laboratories Inc.) as the sole carbon source.

This compound, Mos, consists of a mixture of glucose polymers linked by alpha-1,4-glucosidic bonds. It is devoid of glucose, maltose and maltotriose, and thus consists of higher order oligomers which cannot be used as carbon sources by Saccharomyces cerevisiae strains. This is due to the fact that: 1) these polysaccharides do not enter S. cerevisiae cells and 2) S. cerevisiae strains do not secrete an enzymatic activity (amylase or glucoamylase) capable of degrading Mos extracellularly.

While all three strains transformed with plasmid YEp13 failed to grow on Mos containing medium, the same three strains carrying plasmid pSTA27-1, as well as C. albicans strain 792-1 (courtesy of J. Kwon-Chung, Natl. Inst. Health, U.S.A.) grow well on this medium. This result indicates that the C. albicans DNA fragment cloned in pSTA27-1 carries a gene encoding an enzyme capable of degrading Mos into a usable carbon source.

This gene is expressed in S. cerevisiae, and its product is secreted into the culture medium.

In order to confirm the expression and secretion of the C. albicans glucoamylase by a S. cerevisiae host transformed therewith, the culture media of all six transformed S. cerevisiae strains, as well as that of C. albicans strain 792-1, were tested for the presence of glucoamylase activity according to the method described by Searle et al., FEMS Microbiology, Letters, 111, 211-212 (1981). It was found that while the culture media of S. cerevisiae strains transformed with YEp13 was devoid of any detectable glucoamylase activity, the culture media of C. albicans strain 792-1 and that of S. cerevisiae containing pSTA27-1 contained appreciable amounts of glucoamylase. In fact, it was found that S. cerevisiae strains carrying plasmid pSTA27-1 secrete 15 to 30 fold more glucoamylase activity into their culture medium than does C. albicans strain 792-1.

The glucoamylase activity secreted by S. cerevisiae strains transformed with pSTA27-1 was further characterized and compared to that secreted by C. albicans strain 792-1 in the following manner:

a. The pH optimum of the enzyme secreted by S. cerevisiae strains was determined to be between 4.5 and 6.0 and is identical to that of the enzyme secreted by C. albicans.

b. The temperature optimum of the S. cerevisiae secreted enzyme was found to be 60°C and is identical to that of the C. albicans secreted enzyme.

c. Aliquots of the culture medium derived from an S. cerevisiae strain (genotype: α, leu2, his1,), described above, transformed with either YEp13 or pSTA27-1 and of C. albicans strain 792-1 were analyzed by SDS polyacrylamide gel electrophoresis. A diffuse protein band of a molecular weight higher than 94K dalton is present in the above-described S. cerevisiae strain transformed with pSTA27-1 medium but absent from medium derived from that strain transformed with YEp13. A similar, if not identical, protein band is detected, although in much lower amounts, in medium derived from C. albicans strain 792-1.

Taken together, these results indicate the fragment of C. albicans DNA cloned in pSTA27-1 carries a C. albicans gene encoding a glucoamylase that this gene is expressed in the yeast S. cerevisiae, and that the product of this gene is secreted into the culture medium by S. cerevisiae cells.

Examples 8 and 9 report the isolation and use of several C. albicans replicator regions that support autonomous replication of recombinant plasmids in heterologous host S. cerevisiae microorganisms.


EXAMPLE 8 - TRANSFORMATION OF S. CEREVISIAE WITH A RECOMBINANT PLASMID CONTAINING A C. ALBICANS REPLICATOR REGION


A. Materials and methods

Escherichia coli strain SF$_8$ (C600, leuB, thr, pro, Bl, rk⁻, mk⁻, recBC⁻, lopll, lig⁺) [Cameron et al., PNAS, USA, 72, 3416-3420 (1975)], was used for bacterial transformation and propagation of plasmids. Bacteria were grown on LB (rich media) or M9 (minimal media) supplemented as required [(Miller, "Experiments In Molecular Genetics", Cold Spring Harbor Laboratory, New York, 431-433 (1972)]. Saccharomyces cerevisiae strain 483 (genotype: a, leu2-3, leu2-112, his4-519, canla) was used as host for yeast transformation. This strain was obtained from Michael Culbertson, University of Wisconsin, Madison, Wisconsin. Yeast cultures were grown on YPG (rich media; 1% yeast extract, 1% peptone, 2% glucose) or YNB (minimal medium; Difco YNB) supplemented with histidine (2 μg/ml) and leucine (2 μg/ml) as required.

Plasmid M851 [Gorman et al., Mol. Gen. Genet., 183, 306-313 (1981)] was employed. C. albicans strain 792-1 was used as the source of genomic DNA.

Covalently closed circular plasmid DNA was prepared from M9-glucose grown E. coli cultures amplified with chloramphenicol. DNA was extracted and purified by a cleared lysate technique essentially as described by Clewell, J. Bacteriol., 110, 667-676 (1972).

Crude E. coli plasmid preparations, used for yeast transformation and preliminary gel analysis, were made using the procedure of Birnboim and Doly, Nucl. Acids Res., 7, 1513-1523 (1979). Ten ml of bacterial culture ($2 \times 10^{10}$ cells) yielded 1-2 $\mu$g of crude plasmid DNA. For rapid screening of bacterial transformants for the presence of plasmid, single colony mini-lysates, prepared by the same method, were used.

For recovery of total DNA from S. cerevisiae, the small scale method of Sherman et al., cited above, was employed.

C. albicans DNA was prepared by the method of Rigby et al., J. Mol. Biol., 113, 237-251 (1977).

All gel analysis and hybridization procedures employed were according to the method of Maniatis et al., cited above. E. coli were transformed using the calcium-heat shock method, as modified by Dagert and Ehrlich, Gene, 6, 23-28 (1979). The procedure used for yeast transformation was essentially as described by Beggs Nature, 275, 104-109 (1978), using $\beta$-glucuronidase-sulfatase (Sigma) for spheroplast formation, with the exception that a lower concentration of plasmid DNA (0.5 -1.0 $\mu$g per $10^9$ spheroplasts) was used.

## B. Results

### 1. Plasmid Construction

Total Candida DNA, digested to completion with restriction endonuclease PstI, was mixed with PstI-cut M851 plasmid DNA at concentrations estimated to give maximal recovery of plasmids containing Candida inserts by the method of Dugaiczyk et al., J Mol. Biol., 96, 171-184 (1975), and ligated with T4 polynucleotide ligase. This procedure inserted the DNA by ligation into the unique PstI site of M851. The ligated mixture was used to transform E. coli strain SF8 to leucine prototrophy. Leu$^-$ transformants were then screened for ampicillin sensitivity. Interruption of the amp$^r$ gene by an inserted fragment results in loss of drugs resistance. Thus amp$^s$ colonies (5.8% of the Leu$^+$ bacterial transformants) were selected as presumably harboring plasmids containing sequences from Candida. These hybrid plasmids were designated pCA followed by an isolate number.

### 2. Transformation of Yeast

Plasmids containing a yeast replicator sequence transform yeast at high frequency, reflecting autonomous replication of the plasmid in the eucaryotic cell. If the plasmid lacks a functional replicator it also will transform, but at a low frequency. Presumably, this low-frequency stable transformation reflects integration of the plasmid leu2$^+$ gene into chromosomal DNA by homologous recombination. If Candida chromosomal replicators are functional in yeast, hybrid plasmids containing them might be characterized by the ability to transform yeast at high frequency.

Forty-one (41) pCA plasmids were individually tested for their ability to transform leu2 S. cerevisiae to Leu$^+$. Five of the forty-one pCA plasmids tested showed high frequency transformation These results indicate that such plasmids can replicate autonomously in S. cerevisiae.

### 3. Detection of Plasmid DNA in S. cerevisiae

If high frequency transformation indicates the presence of a Candida sequence that allows autonomous plasmid replication, it should be possible to recover covalently closed circular molecules from the unstable Leu$^+$ yeast transformed with DNA from the pCA plasmids.

Total yeast DNA was extracted from parental and transformed yeast strains, electrophoresed in a 1.0% agarose gel, and transferred to nitrocellulose paper. The DNA was then hybridized with P$^{32}$-labelled pBR322 probe. This probe has no homology with yeast chromosomal DNA. The DNA from transformed cultures was transferred to nitrocellulose filters and hybridized with P$^{32}$-labelled pBR322. DNA sequences hybridizing to this probe could be detected in extracts of S. cerevisiae transformed with one of the five pCA plasmids giving high frequency transformation, as well as with control plasmid pYP42 (Gorman et al., cited above).

4. Transformation of E. Coli with Yeast Plasmid DNA

The yeast plasmid preparations analyzed by hybridization techniques were also examined for ability to transform leuB, E. coli to Leu⁺. Bacterial colonies selected on leucine deficient medium were screened for the presence of plasmid by agarose gel electrophoresis of mini-lysates. The five yeast extracts that were shown to contain plasmids by hybridization have rise to Leu⁺ E. coli that contained plasmid DNA. Plasmid DNa of one of the five high frequency transformation pCA plasmids was compared with the plasmid isolated from E. coli following transfer through the S. cerevisiae host. The plasmids, containing a 4.0 kilobase pair (kbp) C. albicans insert, were found to be identical.

Other replicator regions of Candida useful in the autonomous maintenance of plasmids used to transform Saccharomyces can be isolated according to the method of Kawamura et al., cited above; Hsu et al., cited above; or Tikhomirova et al., cited above. Plasmids containing such replicator regions can be prepared according to the method described in this Example.

## EXAMPLE 9 - TRANSFORMATION OF S. CEREVISIAE WITH A PLASMID CONTAINING A C. ALBICANS REPLICATOR REGON AND A C. ALBICANS CODING SEQUENCE

A plasmid containing a C. albicans replicator region and a C. albicans coding sequence can be prepared by combining the methods of Examples 1 and 8. Such a plasmid can be used to transform S. cerevisiae according to the method of Example 1, and expression of the C. albicans coding sequence by the transformed S. cerevisiae host can be detected according to the method of Example 1.

Genes for glucoamylases from mouse (Thomsen, 1983, Carlsberg Res. Commun., 48:545), wheat, Asperigillus (Innis et al., 1985, Science, 228:21), and S. diastaticus (Yamashita and Fukui, 1983, Agric. Biol. Chem., 47:2689) have been shown to be secreted into the medium when expressed in S. cerevisiae. When produced by C. albicans or by a transformed S. cerevisiae host, the C. albicans enzyme glucoamylase is excreted into the medium. It has now been found that the C. albicans glucoamylase gene, also called the STA gene, isolated as described in Example 7 provides DNA sequences for a novel promoter and a signal peptide which functions in protein secretion. The use of a signal peptide for secretion of a cytoplasmic protein is disclosed, for example, see Silhavy, et al., U.S. Patent 4,335,336 issued June 22, 1982. This glucoamylase gene signal peptide can be used in a system for secretion of native or heterologous proteins expressed by Candida, Saccharomyces or other yeast cells transformed therewith. This expression system targets the heterologous protein to the secretion pathway leading to post- or co-translational modifications of the synthesized protein.

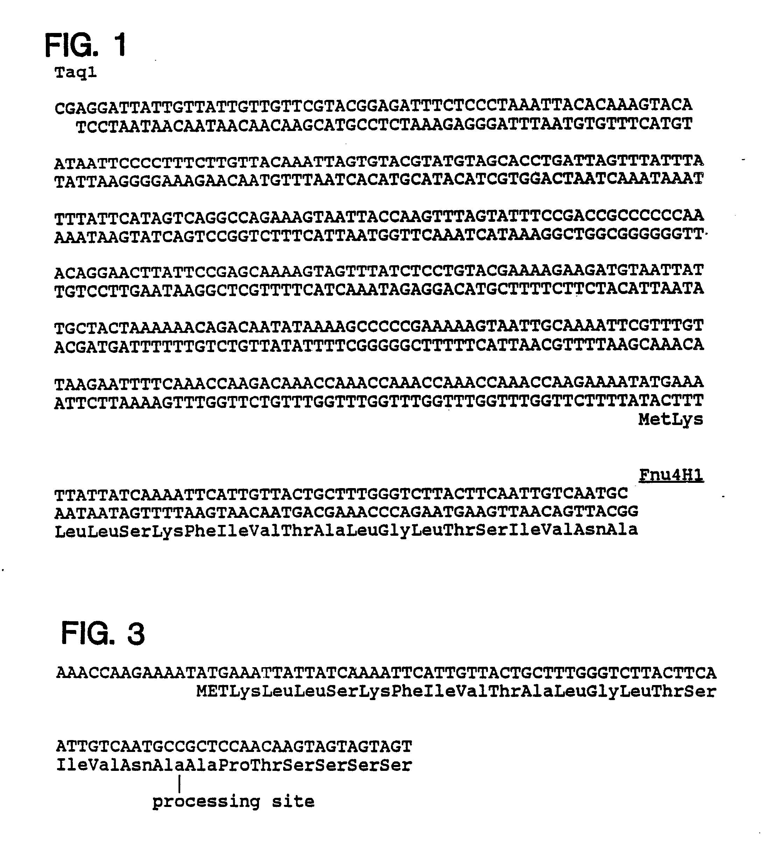

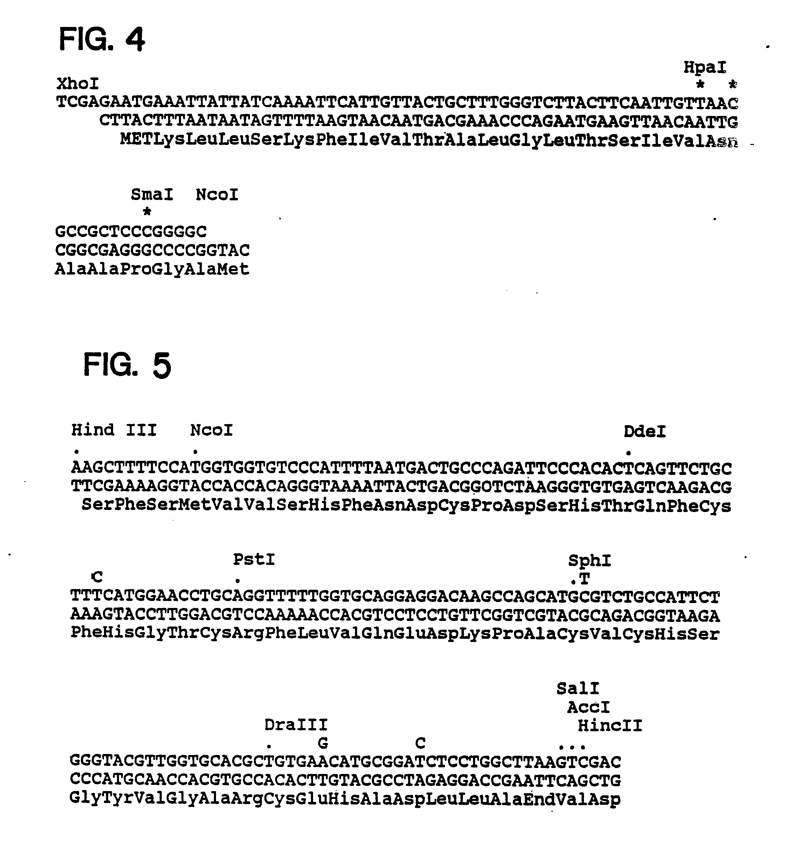

Both the promoter and signal sequences of C. albicans glucoamylase gene are identified in Fig. 1. Fig. 3 illustrates the signal peptide alone. The signal sequence enables the targeting of the heterologous protein to the secretion pathway. Thus, it may be used in an expression system to enable secretion of a recombinant protein into the culture medium whether under control of its own promoter or under control of heterologous yeast promoters.

The following Examples 10-13 demonstrate a variety of uses of this functional DNA sequences of Candida albicans, e.g. the use of the C. albicans promoter and signal sequence to express and secrete a heterologous protein in S. cerevisiae and the use of the C. albicans signal sequence with other yeast promoters for such expression and secretion.

In general in these examples E. coli vectors are constructed which allow inframe translational fusion of heterologous proteins or peptides to the promoter/signal peptide region of this glucoamylase gene. These hybrid gene constructions, (e.g., glucoamylase promoter and signal peptide regions or the signal peptide under control of another yeast promoter) fused to a heterologous gene are then transferred preferably into E. coli/Saccharomyces shuttle plasmid vectors for transformation into yeast cells.

## EXAMPLE 10 - EXPRESSION AND SECRETION OF HIV-I GP160 UNDER CONTROL OF C. ALBICANS GLUCOAMYLASE PROMOTER/SIGNAL PEPTIDE REGION IN S. CEREVISIAE

An expression system employing the C. albicans glucoamylase promoter and signal peptide has been employed to produce the viral envelope glycoprotein gp160 of the HIV-I virus. gp160 is one of the antigens of interest for use in potential AIDS vaccines and therapeutics and can be used as a diagnostic agent for detection of HIV infection.

### A. Construction of E. coli plasmid vectors

E. coli plasmid pUC13 [J. Viera et al., Gene, 19:259-68 (1982)] was linearized by co-digestion with restriction enzymes Accl and HindIII. The linearized plasmid was ligated to a Tag1 to HindIII DNA fragment constructed as follows: A Tag1 to Fnu4H1 DNA fragment comprising the promoter and signal peptide region of the yeast Candida albicans STA gene (encoding the secreted enzyme, glucoamylase) was purified from the cloned gene. The nucleotide sequence of this DNA fragment is shown in Fig. 1.

A synthetic double stranded oligonucleotide having the following sequence:

<div align="center">

**Fnu4H1**         **Bg1II**         **HindIII**

CGCTCCAGATCTA

CGAGGTCTAGATTCGA

</div>

was ligated to the Tag1 to Fnu4 H1 fragment described above. The product of this ligation, (a Tag1 to HindIII DNA fragment) after gel purification, was itself ligated to the Acc1/HindIII digested pUC13 DNA. The ligation mixes were used to transform E. coli strain MM294 (available from the American Type Culture Collection as ATCC 33625 or ATCC 39607).

Transformants were selected on LB plus ampicillin plates and analyzed by colony filter hybridization using a radiolabelled probe containing sequences homologous to the Tag1 to Fnu4H1 fragment. Plasmid DNA from appropriate transformants was subjected to restriction enzyme and nucleotide sequencing analyses. The selected plasmid resulting from these analyses pTI-18 (pRIT13046) contains the entire promoter region of the yeast STA gene, as well as the DNA sequence coding for the first 22 NH₂ terminal amino acids of the glucoamylase protein, which represent the signal peptide of this secreted protein. Immediately following the signal peptide cleavage site, a unique Bg1II restriction cleavage site has been introduced as part of the synthetic oligonucleotide described above.

Cloning of the coding sequence (or part thereof) of foreign genes inter the Bg1II site in such a way as to respect the reading frame of both the signal peptide as well as that of the foreign protein permits once the construct is transferred into a yeast plasmid and introduced into yeast cells, the expression of the hybrid gene via the glucoamylase STA gene promoter and the targeting of the foreign protein to the secretion pathway via the glucoamylase signal sequence.

### B. Cloning of the HIV envelope glycoprotein (gp160) coding sequence in a yeast vector

A genomic clone of the HIV variant BH10 [L. Rattner et al., Nature, 313:277-284 (1985)] was digested with restriction enzymes Asp718 and Xbal. An Asp718 to Xbal DNA fragment of approximately 2550 base pairs was gel purified. This fragment encompasses the coding sequence of the envelope glycoprotein gp160 (the env gene), starting at amino acid Val 42 and extends several hundred nucleotides beyond the translational stop codon (TAA) of the env gene. This fragment was treated with Mung bean nuclease according to Maniatis et al., cited above to render it blunt ended.

Plasmid pIT-18 (pRIT13046) described above was linearized by digestion with restriction enzyme Bg1II, treated with Klenox polymerase I in the presence of deoxynucleotide triphosphates (dNTP's) in order to fill in protruding 5′ ends. The fragment and plasmid were ligated using T4 DNA ligase. E. coli cells were transformed with the ligation mix. Transformants were analyzed by colony filter hybridization. Restriction enzymes and DNA sequence analysis was performed on plasmid DNA extracted from selected transformants.

These analyses allowed the selection of transformants containing plasmid pTI-18/gp160 (pRIT13047). This plasmid contains the HIV env gene fragment defined above, fused in frame to the STA gene signal sequence and preceded by the STA gene promoter region. The DNA and amino acid sequence of the relevant junction between the signal sequence coding region and the env gene coding sequence is provided below.

| GCC | GCT | CCA | GTA | CCT |
|-----|-----|-----|-----|-----|
| Ala | Ala | Pro | Val | Pro |
|  ↑  |     |  ↑  |     |     |

glucoamylase signal BglIII/Asp 718 junction
peptide cleavage site

The relevant part of plasmid pTI-18/gp160 (pRIT13047) was transferred into a yeast vector in the following way. The plasmid was digested with restriction enzymes XbaI and EspI. An XbaI to EspI DNA fragment of approximately 2950 base pairs was gel purified. This fragment contains (starting at the XbaI site and proceeding, towards the EspI site) the following elements:

a few base pairs derived from the polylinker sequence of plasmid pUC13; 355 base pairs representing the STA gene promoter; 66 base pairs coding for the signal peptide of the glucoamylase protein; the coding sequence of the env gene described earlier (starting at Val 42 and ending at its natural TAA translational stop codon), followed by 70 base pairs of untranslated 3' flanking sequences.

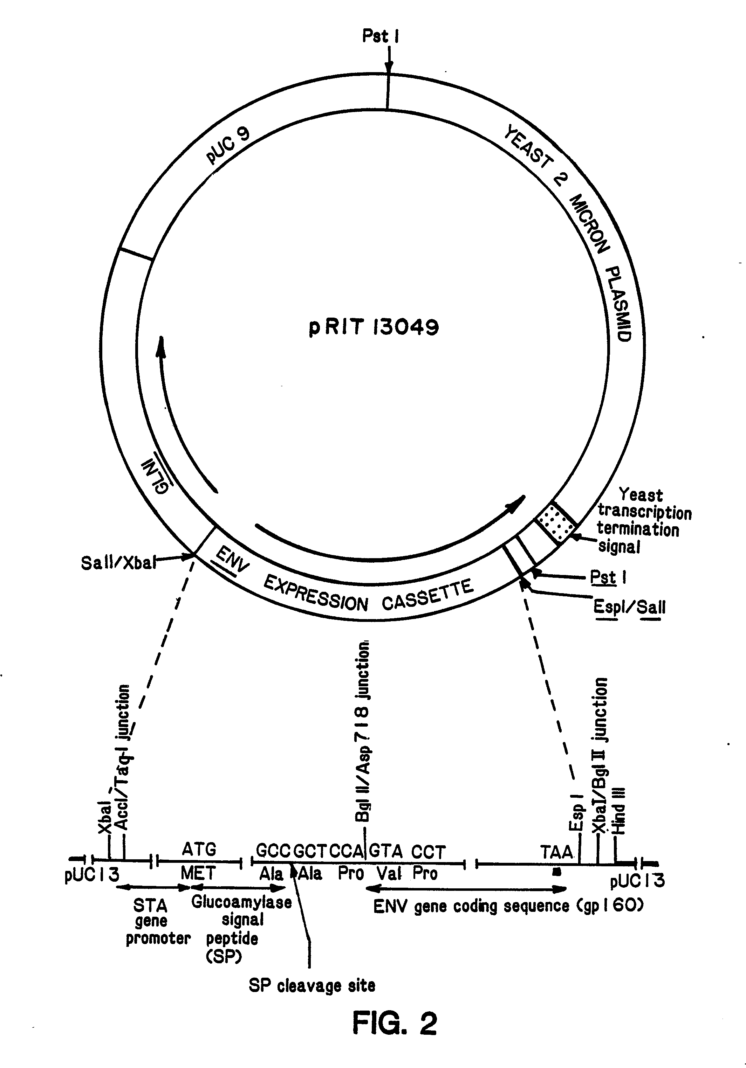

This DNA fragment was treated with the Klenox polymerase I enzyme in the presence of dNTP's to fill in 5' protruding ends. The fragment was ligated to a yeast vector containing the components shown in Fig. 2 except for the expression/secretion cassette for the env gene, previously digested with SalI and treated with Klenox polymerase I and dNTP's. The ligation mix was transformed into E. coli. Ampicillin resistant transformants were screened by colony filter hybridization.

Plasmid DNA extracted from selected transformants was analyzed by restriction enzyme mapping and DNA sequence analysis. Plasmid pQUG3/gp160 (pRIT13049) resulting from this analysis contains the expression/secretion cassette for the env gene immediately followed by a yeast transcription termination sequence derived from the 2μm plasmid gene D [A. Sutton et al., Mol. Cell. Biol., 5:2770-2780 (1985)], and is diameter in Fig. 2. Plasmid pQUG3/gp160 (pRIT13049) was introduced into a S. cerevisiae strain (genotype: Matα, ura3 leu2 adel gln1) by transformation. Yeast transformants were selected for their ability to grow in the absence of glutamine.

C. Expression of the HIV env gene in yeast and localization of the gene product (gp160)

Yeast cells of the S. cerevisiae strain (genotype: Matα ura3 leu2 adel gln1) transformed with plasmid pQUG3/gp160 (pRIT13049) were grown in selective medium. The cells were harvested at late logarithmic or early stationary phase, washed in phosphate buffered saline (PBS) buffer (25 mM NaPO$_4$, 150 mM NaCl, pH 7.4) and resuspended in PBS and 1mM phenylmethylsulfonyl fluoride (PMSF, Sigma) at 1:10 to 1:20 the original culture volume.

Cells were lysed by vigorous vortexing in the presence of glass beads. The broken cell suspension was centrifuged at low speed (1000xg, 10', 4°C) to eliminate unbroken cells and cell debris. The supernatant was retained and represents the crude cell extract (C). An aliquot of fraction C was centrifuged at 30000xg, for 1 hour at 4°C yielding a crude membrane pellet (P1-30) and a supernatant (S1-30) representing the soluble cytoplasmic fraction Fraction P1-30 was resuspended in PBS with 1mM PMSF buffer and extracted with Triton X-100 at a final concentration of 1% detergent. The detergent extracted P1-30 fraction was centrifuged at 30000xg for 1 hour at 4°C. The supernatant fraction (S2-30) was retained. In addition, aliquots of fractions C and S1-30 were extracted with 1% Triton X-100.

The various samples resulting from this cell fractionation scheme were assayed for the presence of material immunologically reactive to antibodies directed against the viral envelope glycoprotein. In this analysis various ELISA sandwich tests were used, utilizing a variety of commercially available polyclonal and monoclonal antibodies directed against portions of the gp160 protein, e.g. the external gp120 glycoprotein and/or its internal gp41 moiety. In some assays, human sera derived from AIDS patients were also used. Material reacting with both anti gp120 as well as anti gp41 antibodies was detected in fraction C (crude cell extract) only following detergent extraction, indicating expression of the env gene and synthesis of gp160 by the tested yeast strains and suggesting association of the synthesized protein with cellular lipids and/or membranes. Approximately 80% of this cross reactive material is recovered in the detergent extracted solubilized membrane fraction (S2-30) confirming the association of this material with cellular lipids and/or membranes. The reactivity of this material to both anti-gp120 as well as anti-gp41 antibodies suggests that most if not all of it is in the form of unprocessed gp160 precursor protein. Analysis of this material by immunoblotting confirms this hypothesis.

### D. Detection of HIV gp160 in a crude membrane extract

Fraction S2-30 was analyzed by capture ELISA for the presence of HIV-env antigenic sites. A sheep antibody to a synthetic peptide of HIV gp41 (Biochrom #D7323) was absorbed to NUNC Immunoplate I microtiter plates at a concentration of $5 \mu g/ml$ in PBS; 50 $\mu$/ml of antibody was incubated overnight at 4°C. The plates were washed by 6 fold replacement of the content of each well with PBS and 0.1% Tween 20 and incubated with 100 $\mu$l of saturation buffer (PBS + 0.1% Tween 20 + 1% bovine serum albumin + 4% newborn calf serum) for 1 hour at 37°C. The plates were then washed as above and the individual wells were incubated with serial dilutions of S2-30 in PBS + 1% Triton. Fifty $\mu$l of antigen were incubated for 2 hours at room temperature. The plates were washed as above and incubated with a monoclonal antibody to HIV gp120 (Dupont #9284/7054). A concentration of 1 $\mu$l/ml of antibody in saturation buffer was used. Fifty $\mu$l well were incubated for 1 hr at 37°C. The plates were washed as described and the binding of the monoclonal anti-HIV gp120 antibody was revealed using biotin labeled anti-mouse immunoglobulin reagent (Amersham RPN 1021) and streptavidin-peroxydase complex (Amersham RPN 1051), according to the manufacturer's instructions. The titration curves obtained indicated the presence of HIV env gene antigenic sites.

### E. Optimization of HIV gp160 yield

The protein gp 160 was produced by batch fermentation of the S. cerevisiae strain (genotype: Matα ura3 leu2 adel gln1) containing plasmid pQU63/gp160 (pRIT13049) lasting 25-30 hours in a standard stirred tank vessel maintained at 30°C. The rich medium (1% yeast extracted 2% peptone, 2% galactose - YEP) was maintained at pH 5.0 by base addition. The inoculum (10% v/v) was grown up for 24 hours on YEP and 2% glucose at 30°C.

Yeast growth was rapid and was measured by reference to its Optical Density ($O.D._{G50}$) at 650nm. The maximum doubling time during exponential phase was approximately 3 hours.

During the exponential growth phase, gp160 was synthesized rapidly and was measured after approximately 12 hours of growth using capture ELISA as described above. Synthesis reached a maximum of 600 $\mu g/ml$ of fermentation broth at approximately 20 hours, whereupon a rapid decline in gp160 was detectable.

The onset of the decline gp160 concentration coincided with the depletion of substrate galactose. Galactose metabolism was followed indirectly by mass spectrometric measurement (each 20 seconds) of the $CO_2$ and oxygen concentration in the fermentor off-gas. The exhaustion of galactose was correlated with the rapid decline of % $CO_2$ in the fermentor off gas at approximately 20 hours.

Thus, harvesting the culture shortly before the decline of % $CO_2$ in the fermentor may be crucial for obtaining a good yield of gp160 in S. cerevisiae.

### F. Purification of gp160 and micelle preparation

One volumes of transformed yeast cells were ground in a Dynomil apparatus in the presence of one volume of buffer consisting of 25mM phosphate pH 7.0 containing 150mM NaCl, 1mM ethylenediaminetetraacetic acid (EDTA) and 1mM PMSF (Buffer A). The suspension is centrifuged for 10 min. at 1000xg and the pellet consisting of unbroken cells and cells debris is discarded. The supernatant is in turn centrifuged 30 min. at 30000xg and the pellet is extracted with 1% Triton X-100 in the original volume of Buffer A. After centrifugation at 30000xg (30 min) the gp160 containing supernatant is treated with 20% Polyethyleneglycol-400 to precipitate gp160. The PEG pellet is dissolved in 50mM Tris buffer pH 7.2 containing 150mM NaCl and 1% Triton X-100 and supplemented with 1.5 M CsCl and 4M guanidine HCl. This solution is ultracentrifuged in a Titanium 50.2 Rotor during 65 hours at 45000 rpm.

The gp160 containing fractions (detected by capture Elisa as above) are pooled, dialysed against PBS and concentrated on an Amicon SM-50 ultramembrane. Dialysed gp160 fraction is then layered on a 10-40% Sucrose gradient and centrifuged in a SW 41 Rotor at 40,000 rpm overnight. The micelle containing fractions that become Elisa positive after Triton treatment are pooled and used for immunization studies.

This yeast synthesized HIV-gp160 envelope glycoprotein obtained via the glucoamylase expression/secretion system of the present invention has potential as a vaccine candidate. The post- or co-translational modifications allowed by this expression system effect the tertiary structure of the protein, which may, in turn, effect the induction of a relevant immune response, since a significant part of the antibody is directed to conformational epitopes. The high cysteine content and the frequent occurrence of potential N-linked glycosylation sites in gp160 suggests the formation of disulfide bridges and the further modification of antigenic properties of the molecule by glycosylation. Thus targeting of gp 160 to the secretion pathway in an expression system using the glucoamylase signal peptide yields a molecule that appears to be structurally and antigenically more related to the native envelope glycoprotein spike. Those structural features of the env-gp160 glycoprotein obtained via the expression system of the present invention are lacking in the envelope protein expressed via non-secretion systems in yeast [see, e.g., P.J. Barr et al., cited above] or in E. coli [see, e.g., S.D Putney et al., Science, 234;1392-96 (1986)].

For example, in this expression system, the yeast synthesized gp160 remains unprocessed into its gp120 and gp41 glycoprotein components. Thus, both proteins remain membrane associated. This is unlike the situation reported in mammalian cells [see, e.g., M.P. Kieny et al., Biotechnology, 4:790-795 (1986)] where the cleavage between gp41 and gp120 results in dissociation of the two molecules and release of most gp120 molecules in the culture supernatant. The association to cell membranes via a hydrophobic transmembrane region of gp41 can serve for the incorporation into particulate structures such as micelles, into liposomes by association to lipids or into immunostimulating complexes (ISCOMS) by association to the detergent quil A. This can increase the immunogenicity of the molecules, both by the adjuvant properties of liposomes and ISCOMS and by allowing an oriented presentation at the surface of these structures, similar to the viral surface.

EXAMPLE 11 - EXPRESSION AND SECRETION OF TGF-α

A. Construction of basic yeast expression vectors

The plasmids used to construct vectors for TGF-α secretion using the glucoamylase signal sequence are based on the high copy yeast shuttle vector pYSK102 [Rosenberg et al., Genetic Engineering, 8:151 (1986)]. All contain an 823 bp EcoRI-PstI fragment of the yeast trpl gene [Tsumper and Carbon, Gene, 10:157 (1980)], which provides a selectable marker, a 2.0 kb EcoRI-PstI fragment of the yeast 2-micron circle, [Hartley and Donaldson, Nature, 286:860 (1980)] which provides an origin of replication in yeast, a portion of pBR322 [in "Cloning Vectors", Pouwels, Enger, Valk, Eds., (1985)] which provides for replication and selection in E. coli and a yeast promoter-terminator module inserted between the HindIII and BamHI sites of pBR322. The pB322 sequences between BamHI and PvuII have been deleted.

The promoter employed in these constructs is either a 431 bp BamHI-RsaI fragment of CUP1 [Karin et al., PNAS USA, 81:337 (1984)], derived as a BamHI-EcoRI fragment from pYSK12 [Butt et al., Proc. Natl. Acad. Sci. USA, 81:3332 (1984); Rosenberg et al., supra; Gorman et al., Gene, 48:13 (1986)] or a 1.1 kb TDH3 promoter sequence [Holland and Holland, J. Biol. Chem., 255:2596 (1980)], derived as a BamHI-EcoRI fragment from plasmid pYSK18 [Rosenberg et al., supra; Gorman et al., supra]. The transcriptional terminator is a 361 bp KpnI-HindIII of the (CYC1 gene [Smith et al., Cell, 16:753 (1979)].

A synthetic linker, with the sequence 5′ GAATTCTCGAGCCATGGCCAGTCGACGTAGTTAAGTAGGTACC 3′ is present in the region between the promoter and the CYC1 termination fragment. This synthetic sequence contains the unique restriction sites XhoI, NcoI and SalI upstream of translational stop codons in all three reading frames.

B. Construction of pYSK140 and pYSK142

The site of signal cleavage of the glucoamylase gene was determined by amino terminal amino acid sequencing of the secreted glucoamylase of Example 7. The DNA sequence of the 5′ end of the glucoamylase gene coding region and the corresponding amino acid sequence, with the site of processing, is shown in Fig. 3. A synthetic oligonucleotide sequence corresponding to the signal sequence and flanking nucleotides containing restriction enzyme sites was synthesized. This sequence is shown in Fig. 4. Asterisks above the bases indicate charges made to introduce restriction endonuclease cleavage sites. The signal sequence ends after the last asterisk. This double standard fragment was inserted in the basic vectors described above which had been cleaved with XhoI and NcoI to give pYSK140 (CUPl promoter) and pYSK142 (TDH3 promoter).

18

The current synthetic glucoamylase signal sequence contains the codons for the amino acids Asn Ala Ala Pro at the 3' end to provide the processing site (see Fig. 3). The presence of the two amino acids Ala and Pro at the 3' side of the cleavage site may be essential for proper cleavage. However, this results in the presence of two extra amino acids on the 5' end of any protein secreted by utilization of this signal sequence. Plasmids are being constructed which will allow fusions directly after the Asn Ala codons. Secretion and processing at the 5' end of fusion proteins made with these vectors are expected to be as useful as the present signal sequence.

C. Construction of pYSK151 and pYSK152

A synthetic oligonucleotide corresponding to the CDNA sequence of the mature human transforming growth factor (TGF-α) [Derynck et al., Cell, 38:287 (1984)] plus additional bases at the 5' and 3' end was synthesized and four conservative base changes made to provide restriction endonuclease sites. This sequence is shown in Fig. 5. The NcoI to SalI portion of the TGF-α sequence was inserted into NcoI and SalI cleaved pYSK140 and pYSK142 to five pYSK151 and pYSK152 respectively. The resulting construction provides an initiating ATG codon at the start of the glucoamylase signal, and an in frame ATG (Met) codon at the start of the TGF-α peptide.

D. Expression and secretion of TGF-α in yeast cultures

Plasmid DNA (2-10 μg/ml) was used to transform S. cerevisiae strain F762 (Mata, trpl 1, ura3-52) using standard techniques [Sherman et al., Methods in Yeast Genetics, Cold Spring Harbor, (1986)], selecting for tryptophan prototrophy. Strain F762 was obtained from Phillip Hunter, Department of Molecular Biology and Genetics, Johns Hopkins Medical School, Baltimore, Maryland. Cultures of selected transformants were grown in YNB (Difco) containing 2% glucose and uracil (20 μg/ul). For plasmids utilizing the CUP1 promoter, copper sulfate at 100 micromolar was included. Cultures were harvested in late log or stationary phase. The cells were spun, and both the culture medium and cells retained. The cell pellets were washed, broken with glass beads in 0.05M acetate buffer, pH 5.0, and then the suspension centrifuged at 5000xg to remove the glass beads and cell debris. The soluble cell extracts and the culture fluids were then dialysed against 2% acetic acid overnight, using tubing of 2,000 molecular weight cut-off.

E. Biological assay of TGF-α

The dialysed cell lysates and culture fluids were analysed for TGF-α activity by three methods.

(1) TGF-a/EGF Radioreceptor Assay

A431A human epidermoid carcinoma cells (1x10^5) were plated overnight in 24-well tissue culture dishes in DMEM containing 10% FCS at 37°C in 10% CO atmosphere. Cells were washed 3 times with binding buffer (Hank's balanced salts solution, 1 mg/ml bovine serum albumen (BSA) and 10 mM Hepes, pH 7.4) at 4°C. Binding buffer (100 ul) containing 1 nm $^{125}$I TGF-α and various concentrations of the sample to be tested or epidermal growth factor (EGF) standard were added simultaneously and incubated at 4°C for 2 hours. Cold competition was performed with a 1000-fold excess of EGF. TGF-α was iodinated using the chloramine T procedure. Cells were washed with ice-cold binding buffer without BSA. The cells were solubilized for 30 min at 37°C with 0.5 ml of 0.5M NaOH. Radioactivity bound was determined using a gamma counter.

(2) Anchorage independent growth assay

Normal rat kidney fibroblasts (NKR-49F) indicator cells (5000) in 0.8 ml of minimal essential medium (DMEM) containing 10% fetal calf serum (FCS) and 0.5% Bacto-agar (Difco) were mixed with aliquots of experimental samples which had been lyophilized, then dissolved in buffer containing 4mM HCl and 2 mg/ml BSA. The cells were added to an 0.8 ml bottom layer of DMEM containing 10% FCS and 0.6% agar in 35 mm tissue culture dishes (Costar, Cambridge, Mass.). Plates were incubated for 8-10 days in 10% CO atmosphere at 37° C. Colonies formed were stained with p-iodonitrotetrazolium violet overnight and counted on a Bausch and Lomb Image Analysis. System programmed to detect colonies greater than 60 um in diameter.

### (3) Radioimmune assay

Assays were performed using a commercially available TGF-α RIA kit (Biotape), according to the manufacturers instructions.

### F. Activity in TGF-α produced in Yeast

The activity of TGF-α produced by plasmid pYSK151 and pYSK152 in shake flask cultures harvested 12 hours after reaching stationary phase was determined. The maximal yield obtained in the medium was approximately 2 mg/liter at $OD_{540} = 2.0$. Greater than 95% of the total activity was found in the medium as compared to the cell extract.

TABLE 1

| Estimated TGF-α Activity (ug/$10^8$ cells) | | |
|---|---|---|
| Plasmid | Medium | Cell extract |
| EGF competition binding assay: | | |
| pYSK151 | 2.87 | 0.143 |
| pYSK152 | 2.70 | 0.135 |
| Soft Agar Assay: | | |
| pYSK151 | 0.718 | 0.006 |
| pYSK152 | 0.617 | 0.005 |
| Radioimmune Assay: | | |
| pYSK151 | 2.31 | |
| pYSK152 | 2.24 | |

### G. Purification of TGF-α

Fermentation broth was centrifuged to remove cells, then filtered. The pH of the filtrate was adjusted to pH 2.7 using glacial acetic acid, then pumped through a column of Whatman Partisil ODS-3, (column dimensions: 48 x 650 mm packing 40-60 μm) at a rate of 200 ml/min which had been equilibrated to 0.1% trifluoroacetic acid (TFA). After loading, the column was washed with 2 column volumes of 0.1% TFA, and 5% i-propanol/0.1% TFA each. Crude TGF-α was eluted with 40% i-propanol/0.1% TFA.

The eluate (4L) was concentrated in vacuo to remove the isopropanol, and then lyophilized. The powder (approximately 600 mg) was redissolved in 3 ml of 1.0 M acetic acid then centrifuged. The supernatant was loaded on to a Biogel P-10 column (2.6 x 120 cm) and eluted with 1.0 M acetic acid at 400 μl/min. Fractions were analyzed by SDS-PAGE electrophoresis. Those fractions containing TGF-α were pooled together and lyophilized.

The powder (14 mg) was loaded onto a Vydac $C_{18}$ column (10 mm x 250 mm) using a gradient 16% acetonitrile/0.05% TFA to 26% acetonitrile/0.05% TFA in 90 minutes. Fractions were collected and analyzed by HPLC. Recovery was approximately 30% of total activity. Three pools containing protein were made and were submitted for EGF/TGF radioreceptor assay, amino acid composition, and sequencing.

All three proteins were found to be biologically active, and varied in chain length at the N-terminal. The sequences were that of TGF-α (50%), Met-TGF-α (18%), and Gly-Ala-Met-TGF-α (22%). Comparison of amino acid composition and receptor binding assay showed that each had a specific activity of 1.0 EGF equivalents.

## EXAMPLE 12 - EXPRESSION AND SECRETION OF ILI-β AND IL1-a

Human ILI-β is expressed in a variety of cell types as a large (31 Kd) precursor polypeptide which is processed and secreted as a mature, unglycosylated, bioactive 17 Kd carboxyl-terminal fragment. Although ILI-β is normally secreted, it lacks a signal sequence.

With the same methods of Example 11, the signal sequence derived from the cloned glucoamylase gene of C. albicans in combination with either the inducible CUP1 or constitutive TDH3 promoter in plasmids pYSK140 and pYSK142, was used to direct the synthesis and secretion of human recombinant interleukin-1 β in S. cerevisiae. cDNA coding for the authentic mature form of ILI-β (carried on a Ban1 fragment which was blunt-ended with Klenow polymerase) was fused as described in Example 11 to the glucoamylase signal sequence at the unique Sma1 site (see Fig. 4) of the pYSK140 and pYSK142, and transformed into yeast.

Each construct directs the efficient secretion of biologically active ILI-β. Greater than 95% of the protein expressed is extracellular, resulting in accumulated levels of more than 2 mg/l as determined by Western blotting. Integration of a single copy of a TDH3 glucoamylase signal sequence ILI-β expression cassette into the genome at the URA3 locus on chromosome V of S. cerevisiae resulted in a strain (GL46: MATβ leu2-3,2-112 trpl-1 ade2-1 his3-11,3-15 canl-100 ura3 ILI-β) which secretes approximately 6 mg/l of product when grown to stationary phase in shake flasks under nonselective conditions (rich media). The amount of extracellular product obtained can be increased by constructing strains containing multiple integrations and/or by growing cells in fed-batch culture.

The secreted form of IL1-β in yeast consists of approximately equimolar amounts of two protein species (17 Kd and 22 Kd). Pulse-chase experiments performed in the presence or absence of tunicamycin, followed by immunonoprecipitation, reveal that the 22 Kd species is a glycosylated form of the molecule. A potential N-linked glycosylation site resides near the amino-terminus. The reason for the appearance of a mixture of two extracellular forms (glycosylated and unglycosylated) is unclear. Kinetic data indicate that the newly secreted product consists primarily of the 22 Kd glycosylated species which may then be converted, via either proteolytic cleavage or by an endoglycosidase, to the 17 Kd form. Direct fusion of the IL1B sequence to the CUP1 inducible promoter without a signal sequence results in expression of the 17 Kd intracellular form, plus a single 17 Kd extracellular species which accumulates in the medium of low phase cultures.

Purified extracellular IL1-β is presently being sequenced to determine the primary signal peptide cleavage site as well as any secondary sites that may be recognized in yeast.

Using similar methods to those outlined in Example 11, the signal sequence derived from the cloned glucoamylase gene of C. albicans, was used to direct the synthesis and secretion of human recombinant interleukin-1 α in S. cerevisiae.

## EXAMPLE 13 - EXPRESSION AND SECRETION OF B. PERTUSSIS TOXIN S1 SUBUNIT

Pertussis toxin, a promising protective antigen for potential use as a vaccine against whooping cough, is a hexameric protein composed of five different subunits. One of the subunits (S1) expresses an enzymatic activity, which is responsible for the toxicity of the protein. However S1 without the other subunits is devoid of toxic activities. The expression of the S1 gene and the secretion of the recombinant product should facilitate production and purification of this subunit, without contamination from the other pertussis toxin subunits.

The cistron of the S1 subunit of pertussis toxin was expressed in S. cerevisiae either as a fusion protein. with secreted glucoamylase or as a non fused but processed protein using the glucoamylase signal peptide.

## A. Plasmid constructs and S. cerevisiae clones

A DNA fragment starting at the second amino acid following signal peptide cleavage site of S1 and extending to the natural TAG translational stop codon encoding an almost complete mature S1 protein was fused, in frame, to the signal peptide coding sequence of the Candida albicans glucoamylase gene. The fused coding sequences are preceded by the 5' non coding sequences of the glucoamylase gene including the promoter and transcription initiation sites of the STA gene (see Fig. 1). The expression cassette was transferred to an E. coli/S. cerevisiae shuttle vector containing the elements of the vector of Fig. 2 minus the env expression cassette, yielding plasmid pRIT13072. Plasmid pRIT13072 contains all the components of the vector of Fig. 2, except that the S1 expression cassette replaces the env expression cassette, and the ligation site on the right side of the cassette is an XbaI/Sal site, instead of an EspI/SalI site. pRIT13072 was transformed into a S. cerevisiae strain (genotype: Matα leu2 ura3 ade1 gln1). A transformed yeast clone, 745.1 [pRIT13072], was selected for further work.

A Sau3a DNA restriction fragment encoding 186 amino acids of the S1 protein (amino acids 2 to 187 of the mature protein) was inserted into the coding sequence of the STA gene (see, Fig. 1). The insert was introduced at an engineered BamHI restriction site located near the carboxy terminal end of the glucoamylase protein. The resulting hybrid gene should express a fusion glucoamylase-S1 protein. The entire expression cassette is carried on an E. coli/S. cerevisiae shuttle plasmid vector similar to the one described above. The resulting plasmid, pRIT13073, was introduced into a S. cerevisiae strain (genotype: Matα leu2 ura3 ade1 gln1) and a transformed clone, 745.1 [pRIT13073], was selected and used for subsequent analyses.

## B. Expression

The S1 subunit synthesized in clone 745.1 [pRIT13072] was readily detectable in Western blots of whole cell extracts using anti-pertussis toxin monoclonal antibody B2F8. The electrophoretic mobility of the recombinant protein in SDS-PAGE appeared to be similar to the mobility of the S1 subunit purified from Bordetella pertussis holotoxin, indicating that the glucoamylase signal peptide is removed and the S1 subunit processed in the yeast cell. Signal peptide cleavage usually occurs during or after translocation of the synthesized protein through the endoplasmic reticulum membrane. The processed S1 subunit should therefore be associated with the crude membrane fraction of yeast cell extracts and/or secreted into the culture medium. Yeast cell extracts were fractionated by differential centrifugation and the different fractions analyzed for the presence of S1. The S1 subunit was associated exclusively with the insoluble material after a 30000 xg centrifugation of the extracts. The pelleted fractions could be solubilized with 1% SDS or 4M urea. A number of other detergents [sodiumdeoxycholate (DOC, Sigma), Triton X100, 3-[(3-cholamidopropyl)-dimethyl ammonio]-1-propane sulfonate (CHAPS, Sigma) and zwitergen] were tested in an attempt to extract the S1 protein from yeast membrane. None had any significant effect on S1 solubilization.

Negative control and S1 containing cell extracts, before and after fractionation, were analysed for the presence of NAD glycohydrolase activities. None of the fractions contained significant enzymatic activities, but all of them strongly inhibited the NAD glycohydrolase activity of S1. No S1 protein could be detected by Western blot or by enzymatic activity analysis, in the culture medium.

Clone 745.1 [pRIT13073 was analyzed for the presence of a glucoamylase-S1 fusion protein (i.e., S1-GA protein). Glucoamylase enzymatic activity was tested by measuring the amount of glucose liberated from the substrate maltotriose at 37°C, in a reaction buffer containing limit NaPO₄, 1% maltotriose, pH 6.5. S1 (NAD-glycohydrolase) activity was measured by the amount (pmoles) nicotinamide released per minute per ml of medium. The parent vector containing no expression cassette showed no detectable glucoamylase or S1 activity in culture medium or extracts. The parent vector containing just a glucoamylase expression cassette (i.e. the STA gene) showed glucoamylase activity in both culture medium and cell extracts, but no S1 activity. In contrast, the yeast strain carrying pRIT13073, the vector containing the glucoamylase S1 fusion expression cassette showed glucoamylase activity in both medium and extract and S1 activity in medium. Thus, S. cerevisiae cells transformed with plasmid pRIT13073 synthesize and secrete into the culture supernatant proteins that express glucoamylase and S1-specific NAD-glycohydrolase activities. This result suggests that the enzymatically active portion of S1 was successfully fused to glucoamylase, synthesized in yeast and secreted into the culture supernatant.

These results show that the S1 subunit of B. pertussis toxin can be synthesized in yeast under the control of the glucoamylase or STA gene promoter, secreted into the yeast membranes via the glucoamylase signal peptide or exported into the culture medium as a fusion protein with glucoamylase. In the latter case (i.e. the fusion glucoamylase-S1 protein) the S1 subunit is enzymatically active.

C. Immunoprotective activity

Two mice were each injected with 35 μg S1-GA(pRIT13073) (the S1-glucoamylase fusion protein prepared in Example 13, part B from the transformation of S. cerevisiae with pRIT13073) or glucoamylase (GA) in CFA, at day 0, and twice in IFA at days 30 and 58. The GA and S1-GA(pRIT13073) fusion protein used were both synthesized in S. cerevisiae and partially purified from the culture supernatant in a non-denaturing manner, such as by DEAE-cellulose chromatography, prior to the injection into mice. The mice were bled at days 54 and 75. The sera were analyzed by ELISA for the presence of anti-PT, anti-S1 and anti-rSld antibodies, and by the Chinese Hamster Ovary (CHO) Cell assay (Hewlett et al. (1983) Infect. Immun. 55, 24-38) for the presence of PT neutralizing antibodies. All mice injected with S1-GA(pRIT13073) fusion protein had increased anti-PT, anti-S1 and anti-rSld antibody titers. Antibody titers against these antigens did not significantly increase in the mice immunized with GA. All mice injected with S1-GA-(pRIT13073) also had increased CHO cell neutralization titers. The arithmetic mean value was 920 for S1-GA(pRIT13073). These results show that the S1-GA protein is sufficient to induce a neutralizing and protective response, and that such protein is useful in a vaccine against pertussis.

In a manner similar to the method outlined in Example 13, parts A and B, the Sau3a DNA restriction fragments encoding 186 amino acids of the S1 protein (amino acids 2 to 187 of the mature protein) could be fused, in frame, to the signal peptide coding sequence of the Candida albicans glucoamylase gene, the resulting expression cassette could be transferred to an appropriate vector, the resulting vector could be transferred into an appropriate host S. cerevisiae strain, and the resulting protein could be used in a vaccine against pertussis.

EXAMPLE 14-PARENTERAL VACCINE PREPARATION

5-25 μg of S1-GA(pRIT13073), prepared according to the method of Example 13, is mixed with an aluminum adjuvant, such as aluminum hydroxide, to produce a vaccine in a form appropriate for incorporation into a parenteral administration dosage form.

While the above descriptions and examples fully describe the invention and the preferred embodiments thereof, it is understood that the invention is not limited to the particular disclosed embodiments.

This invention is not limited to the genes and promoters of Candida given in the examples above. Rather, this invention provides a general method for location Candida gene expression units, promoters, regulatory regions and structural genes as well as a general method for expressing such functional DNA sequences in Saccharomyces.

Further, the heterologous genes expressed under control of the C. albicans glucoamylase signal sequence and yeast promoters are not limited to the exemplary proteins described herein. One of skill in the art will acknowledge the use of the described expression system for expressing and secreting a variety of heterologous genes of interest.

Thus, the invention includes all embodiments coming within the scope of the following claims.

**Claims**

1. A transformed host microorganism of the genus Saccharomyces which comprises a functional DNA sequence derived from the same or substantially the same coding sequence as the signal sequence of the C. albicans glucoamylase gene.

2. The microorganism of Claim 1 wherein the functional DNA sequence comprises the same or substantially the same DNA sequence as that of Fig. 3.

3. The microorganism of Claim 1 wherein the functional DNA sequence comprises said signal sequence preceded by a yeast promoter.

4. The microorganism of Claim 1 which comprises a functional DNA sequence the same or substantially the same as the promoter sequence of the C. albicans glucoamylase gene.

5. The microorganism of Claim 4 wherein the functional DNA sequence comprises the same or substantially the same DNA sequence at that of Fig. 1.

6. The microorganism of Claim 1 wherein said signal sequence is fused in proper orientation with a selected heterologous gene.

7. The microorganism of Claim 6 wherein said heterologous gene is selected from the group consisting of TGF-α, Interleukin-1β, Interleukin-1α, S1-GA protein, an HIV-I protein or fragments thereof.

8. The microorganism of Claim 1 which belongs to the species Saccharomyces cerevisiae.

9. The microorganism of Claim 1 which comprises a recombinant plasmid, wherein said plasmid comprises a replicator region and a functional DNA sequence derived from the same or substantially the same sequence as the signal sequence of the C. albicans glucoamylase gene.

10. A recombinant DNA plasmid comprising a functional DNA sequence containing a coding sequence the same or substantially the same as the signal sequence of the C. albicans glucoamylase gene.

11. The plasmid of Claim 10 wherein the functional DNA sequence comprises the same or substantially the same DNA sequence as that of Fig. 3.

12. The plasmid of Claim 10 wherein the functional DNA sequence comprises said signal sequence preceded by a yeast promoter.

13. The plasmid of Claim 10 which comprises a functional DNA sequence the same or substantially the same as the promoter sequence of the C. albicans glucoamylase gene.

14. The plasmid of Claim 13 wherein the functional DNA sequence comprises the same or substantially the same DNA sequence as that of Fig. 1.

15. The plasmid of Claim 10 wherein said signal sequence is fused in proper orientation with a selected heterologous gene.

16. The plasmid of Claim 15 wherein said heterologous gene is selected from the group consisting of TGF-α, Interleukin-1β, Interleukin-1α, S1-GA protein, an HIV-I protein or fragments thereof.

17. A method of expressing a functional DNA sequence comprising a sequence the same or substantially the same as the signal or promoter sequence of the C. albicans glucoamylase gene which comprises transforming a host microorganism of the genus Saccharomyces with the functional DNA sequence, and culturing the transformed host under suitable conditions such that the functional DNA sequence is expressed.

18. The method of Claim 17 wherein said signal sequence is fused, in phase, and in proper orientation, with a selected heterologous gene.

19. The method of Claim 18 wherein said heterologous gene is selected from the group consisting of TGF-α, Interleukin-1β, Interleukin-1α, S1-GA protein, an HIV-I protein or fragments thereof.

20. The method according to Claim 17 wherein the host microorganism belongs to the species Saccharomyces cerevisiae.

21. A promoter sequence for use in yeast expression vectors comprising the same or substantially the same sequence as that of Fig. 1.

22. A signal sequence for use in yeast expression vectors enabling the secretion of heterologous genes fused thereto comprising the same or substantially the same sequence of Fig. 3.

23. An HIV-I glycoprotein gp160 produced in unprocessed form by culturing a transformed Saccharomyces host cell wherein said host cell comprises a gp160 gene sequence fused in phase and in proper orientation to a yeast promoter and the signal sequence of the Candida albicans glucoamylase gene.

24. The glycoprotein of claim 23 produced in micellar form.

25. TGF-α produced by culturing a transformed Saccharomyces host cell wherein said host cell comprises a TGF-α gene sequence fused in phase and in proper orientation to a yeast promoter and the signal sequence of the Candida albicans glucoamylase gene.

26. S1-GA protein produced by culturing a transformed Saccharomyces host cell wherein said host cell comprises a S1-GA protein coding sequence fused in phase and in proper orientation (a) to a yeast promoter and (b) within the Candida albicans glucoamylase gene provided that such fusion includes the signal sequence of the Candida albicans glucoamylase gene.

27. The protein of Claim 26 which is S1-GA(pRIT13073).

28. S1-GA protein produced by culturing a transformed Saccharomyces host cell wherein said host cell comprises a S1-GA protein coding sequence fused in phase and in proper orientation to a yeast promoter and the signal sequence of the Candida albicans glucoamylase gene.

29. IL1-β produced by culturing a transformed Saccharomyces host cell wherein said host cell comprises a IL1-β gene sequence fused in phase and in proper orientation to a yeast promoter and the signal sequence of the Candida albicans glucoamylase gene.

30. IL1-α produced by culturing a transformed Saccharomyces host cell wherein said host cell comprises a IL1-β gene sequence fused in phase and in proper orientation to a yeast promoter and the signal sequence of the Candida albicans glucoamylase gene.

31. A vaccine for stimulating protection against whooping cough wherein such vaccine comprises an immunoprotective and non-toxic quantity of S1-GA protein.

32. The vaccine of Claim 31 which comprises S1-GA(pRIT13073) protein.

33. A method for innoculating a human against subsequent whooping cough which comprises administering the vaccine of Claim 31 to such human.

34. S1-GA protein.

35. S1-GA protein coding sequence.

36. A recombinant DNA molecule comprising the S1-GA coding sequence of Claim 35.

37. A recombinant DNA vector comprising the S1-GA coding sequence of Claim 35.

38. A synthetic DNA molecule comprising the S1-GA coding sequence of Claim 35.

39. A host cell transformed with the S1-GA coding sequence containing vector of Claim 37.

# FIG. 1

Taq1

CGAGGATTATTGTTATTGTTGTTCGTACGGAGATTTCTCCCTAAATTACACAAAGTACA
TCCTAATAACAATAACAACAAGCATGCCTCTAAAGAGGGATTTAATGTGTTTCATGT

ATAATTCCCCTTTCTTGTTACAAATTAGTGTACGTATGTAGCACCTGATTAGTTTATTTA
TATTAAGGGGAAAGAACAATGTTTAATCACATGCATACATCGTGGACTAATCAAATAAAT

TTTATTCATAGTCAGGCCAGAAAGTAATTACCAAGTTTAGTATTTCCGACCGCCCCCCAA
AAATAAGTATCAGTCCGGTCTTTCATTAATGGTTCAAATCATAAAGGCTGGCGGGGGGTT·

ACAGGAACTTATTCCGAGCAAAAGTAGTTTATCTCCTGTACGAAAAGAAGATGTAATTAT
TGTCCTTGAATAAGGCTCGTTTTCATCAAATAGAGGACATGCTTTTCTTCTACATTAATA

TGCTACTAAAAAACAGACAATATAAAAGCCCCCGAAAAAGTAATTGCAAAATTCGTTTGT
ACGATGATTTTTTGTCTGTTATATTTTCGGGGGCTTTTTCATTAACGTTTTAAGCAAACA

TAAGAATTTTCAAACCAAGACAAACCAAACCAAACCAAACCAAACCAAGAAAATATGAAA
ATTCTTAAAAGTTTGGTTCTGTTTGGTTTGGTTTGGTTTGGTTTGGTTCTTTTATACTTT
                                                        MetLys


                                                          Fnu4H1
TTATTATCAAAATTCATTGTTACTGCTTTGGGTCTTACTTCAATTGTCAATGC
AATAATAGTTTTAAGTAACAATGACGAAACCCAGAATGAAGTTAACAGTTACGG
LeuLeuSerLysPheIleValThrAlaLeuGlyLeuThrSerIleValAsnAla


# FIG. 3

AAACCAAGAAAATATGAAATTATTATCAAAATTCATTGTTACTGCTTTGGGTCTTACTTCA
            METLysLeuLeuSerLysPheIleValThrAlaLeuGlyLeuThrSer


ATTGTCAATGCCGCTCCAACAAGTAGTAGTAGT
IleValAsnAlaAlaProThrSerSerSerSer
                |
            processing site

FIG. 2

# FIG. 4

```
                                                                    HpaI
XhoI                                                                 *    *
TCGAGAATGAAATTATTATCAAAATTCATTGTTACTGCTTTGGGTCTTACTTCAATTGTTAAC
       CTTACTTTAATAATAGTTTTAAGTAACAATGACGAAACCCAGAATGAAGTTAACAATTG
       METLysLeuLeuSerLysPheIleValThrAlaLeuGlyLeuThrSerIleValAsn -


             SmaI   NcoI
               *
GCCGCTCCCGGGGC
CGGCGAGGGCCCCGGTAC
AlaAlaProGlyAlaMet
```

# FIG. 5

```
Hind III     NcoI                                         DdeI
  •            •                                            •
AAGCTTTTCCATGGTGGTGTCCCATTTTAATGACTGCCCAGATTCCCACACTCAGTTCTGC
TTCGAAAAGGTACCACCACAGGGTAAAATTACTGACGGOTCTAAGGGTGTGAGTCAAGACG
 SerPheSerMetValValSerHisPheAsnAspCysProAspSerHisThrGlnPheCys


                PstI                                      SphI
  C               •                                       .T
TTTCATGGAACCTGCAGGTTTTTGGTGCAGGAGGACAAGCCAGCATGCGTCTGCCATTCT
AAAGTACCTTGGACGTCCAAAAACCACGTCCTCCTGTTCGGTCGTACGCAGACGGTAAGA
PheHisGlyThrCysArgPheLeuValGlnGluAspLysProAlaCysValCysHisSer


                                               SalI
                                               AccI
                    DraIII                     HincII
                      •      G          C      ...
GGGTACGTTGGTGCACGCTGTGAACATGCGGATCTCCTGGCTTAAGTCGAC
CCCATGCAACCACGTGCCACACTTGTACGCCTAGAGGACCGAATTCAGCTG
GlyTyrValGlyAlaArgCysGluHisAlaAspLeuLeuAlaEndValAsp
```